# EUROPEAN PATENT SPECIFICATION

(11) **EP 2 785 298 B1**
(45) Date of publication and mention of the grant of the patent: **20.06.2018**
(21) Application number: 12798568.7
(22) Date of filing: 30.11.2012
(51) Int. Cl.: A61F 13/00, A61F 13/537, D04H 3/12, D04H 3/14, D04H 3/147, D04H 1/407

(54) **ASSEMBLED INTERMEDIATE COMPRISING A COILED-FILAMENT NONWOVEN WEB AND ARTICLES**
ZUSAMMENGESETZTES ZWISCHENPRODUKT MIT EINEM VLIESSTOFF AUS SPIRALFILAMENTEN UND ARTIKEL
INTERMÉDIAIRE ASSEMBLÉ COMPRENANT UNE BANDE NON TISSÉE À FILAMENTS SPIRALÉS ET ARTICLES

(30) Priority: 01.12.2011 US 201113308936
(43) Date of publication of application: 08.10.2014
(73) Proprietor: 3M Innovative Properties Company, St. Paul, MN 55133-3427 (US)
(72) Inventor: WOOD, Leigh E., Saint Paul, Minnesota 55133-3427 (US); PRIOLEAU, Lori-Ann S., Saint Paul, Minnesota 55133-3427 (US); LALIBERTE, Thomas R., Saint Paul, Minnesota 55133-3427 (US); EHLERS, Kerstin, D-41453 Neuss (DE)
(74) Representative: Vossius & Partner Patentanwälte Rechtsanwälte mbB
(86) International application number: PCT/US2012/067187
(87) International publication number: WO 2013/082355

(56) References cited:
- EP-A1- 1 504 739
- EP-A1- 2 058 424
- US-A- 5 147 345
- US-A- 6 096 015
- US-A1- 2002 010 448

## Description

The invention relates to an absorbent assembled intermediate and an absorbent article.

US 2002/010448 A1 relates to an absorbent article including: a support sheet; a liquid-absorbing layer disposed on the support sheet; and a liquid-permeable surface sheet covering the liquid-receiving face of the liquid-absorbing layer. Between the liquid-absorbing layer and the surface sheet, there is provided a liquid-permeable layer of crimped fibers which remain in a crimped state in a natural state without any external force. The crimped fiber layer is formed by interconnecting the crimped fibers to leave voids therebetween.

EP 1 504 739 A1 relates to an acquisition member for an absorbent article, the acquisition member comprising 20 to 40 weight percent of a latex binder, and 60 to 80 weight percent of polyester fibers, wherein the fibers comprise 20 to 80 weight percent of a first type of fibers, and 20 to 80 weight percent of a second type of fibers, the second type of fibers comprising spiral-crimp fibers.

EP 2 058 424 A1 relates to a nonwoven fabric. The fabric comprises a conjugated fiber comprising a plurality of resins which are different in thermal shrinkage and form a phase separation structure, wherein the conjugated fibers are arranged in a direction approximately parallel to a surface direction of the nonwoven fabric and crimped and the conjugated fibers have an average curvature radius of fiber crimp of 20 to 200 µm, and the crimps are distributed approximately uniformly in a thickness direction of the nonwoven fabric.

### Summary

An assembled intermediate comprising a fluid transport element proximate an absorbent material is described. The fluid transport element comprises a thermoplastic nonwoven web comprising a plurality of coiled filaments having an average diameter of 200 to 500 microns interengaged by means of intermittent bonds. The web has a thickness of 3 to 20 mm, a density ranging from 0.02 to 0.10 g/cm³, and a work of compression of no greater than 20 kJ/m³ according to the compressibility test method.

Also described are absorbent articles, comprising the assembled intermediate described herein, proximate an absorbent material, with the proviso that the absorbent article is not a personal hygiene article. The absorbent article may be suitable for use for spill containment or medical uses, such as wound dressings.

The present invention is defined by the features of the claims.

### Brief Description of the Drawings

**Figure 1** is a schematic of a method of producing a stretched coiled-filament web;
**Figure 2** is a schematic of an embodiment of an apparatus that can be used to make (an unstretched) coiled-filament web;
**Figure 3** is a microphotograph of an unstretched coiled-filament web;
**Figure 4** is a microphotograph of an embodied stretched coiled-filament web;
**Figure 5** is a microphotograph of coiled-filament comprising a broken bond;
**Figures 6-7** are microphotographs of embodied stretched coiled-filament webs;
**Figure 8** is a schematic of an embodiment of an apparatus that can be used to further fabricate a coiled-filament web into assembled intermediates or articles;
**Figure 9** is a microphotograph of an illustrative unstretched coiled-filament web;
**Figure 10** is a microphotograph of an illustrative stretched and relaxed coiled-filament web;
**Figure 11** is a top view of an absorbent article;
**Figure 12** is a cross-sectional view of the absorbent article of Figure 11;
**Figures 13-17** are cross-sectional views of other absorbent articles;
**Figure 18** is a top view of another absorbent article;
**Figure 19** is a cross-sectional view of the absorbent article of Figure 18.

### Detailed Description

Presently described are assembled intermediates comprising a coiled-filament web proximate an absorbent material. The assembled intermediates may be suitable for use in personal hygiene articles or other absorbent articles. Also described are absorbent articles suitable for use for spill containment or medical uses, such as wound dressings, comprising the assembled intermediate. Such absorbent articles are not personal hygiene articles, such as adult incontinent products, sanitary napkins, and disposable diapers.

The filaments of the coiled-filament web are formed from and thus comprise a thermoplastic polymer. Examples of suitable thermoplastic polymers that can be used to form the filaments or components thereof include polymers selected from the following classes: polyolefins, such as polyethylenes, polypropylenes, polybutylenes, blends of two or more of such polyolefins, and copolymers of ethylene and/or propylene with one another and/or with small amounts of polymerizable, higher, alpha olefins, such as pentene, methylpentene, hexane, or octane; halogenated polyolefins, such as chlorinated polyethylene, poly(vinylidene fluoride), poly (vinylidene chloride), and plasticized poly(vinyl chloride); copolyester-ether elastomers of cyclohexane dimethanol, tetramethylene glycol, and terephthalic acid; copolyester elastomers such as block copolymers of polybutylene terephthalate and long chain polyester glycols; polyethers, such as polyphenyleneoxide; polyamides, such as poly(hexamethylenedipamide), e.g., nylon 6 and nylon 6,6; nylon elastomers; such as nylon 11, nylon 12, nylon 6,10 and polyether block polyamides; polyurethanes; copolymers of ethylene, or ethylene and propylene, with (meth)acrylic acid or with esters of lower alkanols and ethylenically-unsaturated carboxylic acids, such as copolymers of ethylene with (meth) acrylic acid, vinyl acetate, methyl acrylate, or ethyl acrylate; ionomers, such as ethylene-methacrylic acid copolymer stabilized with zinc, lithium, or sodium counterions; acrylonitrile polymers, such as acrylonitrile-butadiene-styrene copolymers; acrylic copolymers; chemically-modified polyolefins, such as maleic anhydride- or acrylic acid-grafted homo- or co-polymers of olefins and blends of two or more of such polymers, such as blends of polyethylene and poly(methyl acrylate), blends of ethylene-vinyl acetate copolymer and ethylene-methyl acrylate; and blends of polyethylene and/or polypropylene with poly(vinyl acetate). The foregoing polymers are normally solid, generally high molecular weight, and melt-extrudable such that they can be heated to form molten viscous liquids which can be pumped as streams to the extrusion die assembly and readily extruded therefrom under pressure as the filaments.

Preferably the coiled filaments are tough, durable, melt-bondable, thermoplastic, large-diameter filaments comprising synthetic organic plastic polymer or blends or multicomponent polymer filaments. In some embodiments, the filaments of the web comprise the same synthetic organic plastic material. In some embodiments, the filaments are formed from semicrystalline thermoplastic polymers, such as polyolefins. In other embodiments, a portion of the filaments comprise one type of synthetic organic plastic material and a portion of the filaments comprise a different type of synthetic organic plastic material. The filament can comprise a plurality, e.g., 2 to 5, of components such as bicomponent filaments, such as a sheath-core or side-by-side filaments. The plastics forming the filaments can further have incorporated adjuvants or additives to enhance a property of or impart a property to the filament, such as stabilizers, processing aids, fillers, coloring pigments, crosslinking agents, foaming agents, and fire retardants.

Generally the filaments of the coiled-filament web have an average width, diameter, or cross-section dimension of at least 50 microns (i.e. 0.05 mm), or at least 100 microns, or at least 150 microns, or at least 200 microns. The average diameter may range up to 1000 microns (1 mm), yet is typically no greater than 800 microns, or 700 microns, or 600 microns, and in some embodiments no greater than 500 microns or 400 microns. The filament cross sectional dimension (and shape of the cross section) is preferably substantially, or essentially, uniform along the length of the filament, e.g., uniformly round. The surface of the filament is typically smooth. The filaments can be in the shape or form of fibers, strips, or other narrow and long shapes. Aggregations can be made up of a plurality of filaments with the same or different plastic compositions, geometric shapes, sizes, and/or diameters. The filaments are typically solid. The filaments can be circular or round in cross section or non-circular in cross section, e.g., lobal, elliptical, rectangular, triangular, and shapes with radial arms such as "x-shaped". The filaments are preferably continuous in length, that is, of indefinite length in a common direction (e.g. machine direction).

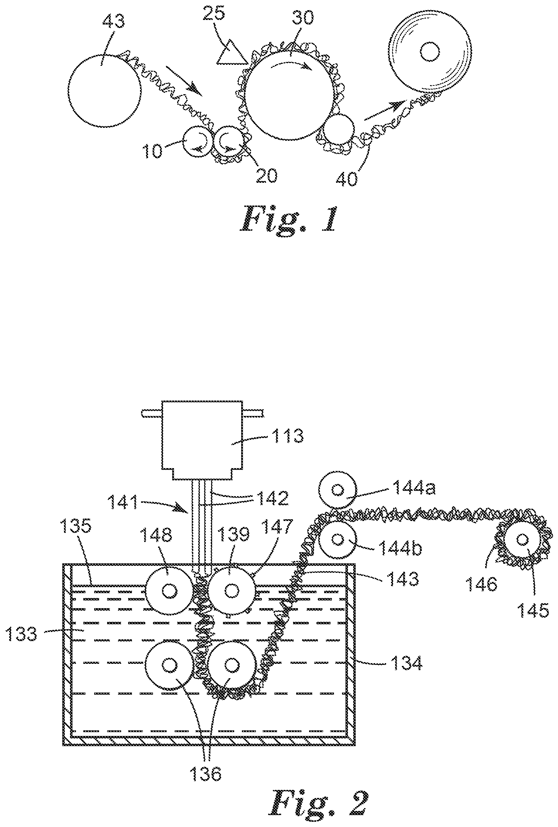

With reference to FIG. 2, a coiled-filament web as can be made by processes described in the art (See for example U.S. Pat. Nos. 4,227,350; 4,351,683; 3,691,004; and 6,762,139).

The filaments are melt-extruded as a bundle or group of free-falling, closely spaced, generally parallel, discrete, continuous, filaments of hot, tacky, deformable, viscous polymer melts, the hot filaments are then convoluted (e.g. coiled) and cooled, or quenched, to a generally non-tacky or non-adhesive solid state. The hot filaments can be cooled by contact with a cooling means or medium, such as a liquid quench bath, e.g., a body of water. The web can then be advanced or conveyed through the bath and withdrawn therefrom. The web is convoluted (e.g. coiled) into discrete continuous filaments as the filaments are conveyed through the quench bath at a take-away speed which is less than the speed of the filaments entering the quench bath. This permits the falling, molten, still deformable filaments to convolute (e.g. coil into an essentially helical shape) adjacent the surface of the quench bath as described in U.S. Pat. No. 4,227,350. The use of a surfactant (for example, as described in said U.S. Pat. No. 3,837,988) in the quench bath may be desirable to aid coil formation.

In one embodiment, the nonwoven webs are generally made with an extruder die assembly 113 that extrudes downwardly a plurality or bundle 141 of hot, continuous, large-diameter filaments 142, that fall freely in the quiescent ambient air into tank 134. The bundle 141 can be aligned so that some of the hot, viscous filaments 142 are permitted to make glancing contact with the outer surface of a guide roll 139. The roll 139 is optionally provided with spaced-apart guide pins or pegs 147, or some other type of guide, such as a stationery plate, to guide the hot, viscous filaments as they move, toward the surface 135 of a body or bath 133 of quench liquid, such as water, in tank 134. The surface of the quench liquid is disposed a suitable distance below the lower face of the extruder die assembly of 113 so as to achieve the desired diameter of the filaments as they enter the bath. The guide roll 139 can be set to cause glancing contact with the filaments 142, as described in said U.S. Pat. No. 4,351,683. As the hot, viscous filaments 142 fall in the ambient air, they begin to cool from the extruding temperature (which can range, for example, from 150°C to 400°C). The extrusion temperature is typically sufficiently higher than the melt temperature such that the filaments coil. The guide roll 139 (as well as optional rolls downstream, e.g. 136, 144a, 144b and 145) can be set to rotate at a predetermined speed or rate such that the rate of lineal movement of the filaments 142 as they enter the body 133 of quench liquid is slower than the rate of linear movement of the hot, viscous filaments upstream of the guide roll(s). Since the take-away speed of the formed web 143 is slower than the speed of the hot filaments entering the quench bath 133, and the filaments 142 are still in a sufficiently viscous, deformable, or molten state, the filaments entangle or aggregate themselves by coiling, undulating, or oscillating just above the surface 135 of the quench liquid 133 into which they enter and can further cool, e.g., to about 50°C, quickly enough so that their shape does not deform, and solidify or rigidify just below the surface 135. A degree of resistance is imparted to the flow or free fall of the hot, viscous filaments 142 about the surface 135 by the already quenched, aggregated filaments in the quench bath 133 (quench liquid and quench bath are both referred to as 133) which causes the still deformable filaments entering the quench bath to coil, oscillate, or undulate just above the surface of the bath. This motion establishes irregular or random periodic contact between the still-hot filaments, resulting in spot- or tack- melt-bonding of contiguous surfaces of the filaments at their points of contact or intersection. Consequently, the filaments 142 assume a coiled, looped, sinuous or undulating configuration and become entangled or inter-engaged. The filaments 142 upon entering the quench liquid 133 and passing adjacent immersed guide roll 139 form an integrated web 143 of intermittently (e.g. spot- or tack-bonded) solidified filaments.

The web 143 can be conveyed and withdrawn from the tank 134 by means of pinch rolls 144a and 144b and wound by roll 145 to form a winding 146 of the web.

The contact surface(s) used may be in motion, as for example the surface of a rotating cylindrical drum, as described in said U.S. Pat. No. 4,351,683, so as to collect the newly forming web and help convey it into and/or through the quench bath. The contact surfaces may alternatively be stationary, as for example, a plate, such as described U.S. Pat. No. 3,691,004. The unified web thus formed comprises overlapping or entangled loops or coils of filaments and has sufficient structural integrity to allow the web to be conveyed, transported, or otherwise handled.

In one embodiment, the method of making the coiled-filament web comprises the steps of simultaneously (or cojointly) melt-extruding thermoplastic polymers through a plurality of extruder die openings or orifices, in the form of a plurality of discrete and separate hot, tacky molten filaments, entangling the filaments into the form of a web, cooling them, for example, in a water quench bath, and recovering the resulting non-tacky, solidified filaments as a web of such filaments. The diameter of the die openings can vary depending on the desired filament diameter. Since the filaments draw to some extent while free-falling, the extruder die openings are typically larger than the final filament diameter. For coiled-filament webs having a (cross-web) width of about 10 cm, the number of extruder die openings may be relatively small (e.g. 50 to 200). However, for larger (cross-web) width webs the number of extruder die opening is typically at least 300, 400, or 500 and typically no greater than 3000. In some embodiments, the number of extruder die openings is at least 20, 25, or 30 openings per linear inch of die.

This process produces a web wherein each filament is coiled and undulated throughout its length. The ratio of the actual filament length (i.e. uncoiled in machine direction) to the coiled filament length is typically at least 2:1 or 3:1 and typically no greater than 8:1. In some embodiments, the ratio of the actual filament length (i.e. uncoiled in machine direction) to the coiled filament length is no greater than 7:1, or 6:1, or 5:1. This ratio is typically constant with unchanged process conditions. Thus, although webs of various ratios can be made, a single web can typically be characterized by a ratio having a small range of actual filament length to coiled filament length. The undulations of each filament are typically irregular. However, it is possible to adjust the process to produce a regular helically coiled filament. Irregular filament undulation can be characterized by random looping, kinking, or bending of the filaments throughout the web in a pattern defined generally by the openings of extruder die. Typically more than one row of filaments are extruded to produce a web having layers of coiled and undulated filaments, each layer representing a row of extruded filaments. Each layer can be discernible in the web, sometimes with great difficulty. The adjacent filaments between layers are typically bonded together where the filaments contact one another, thereby forming intermittent melt bonds.

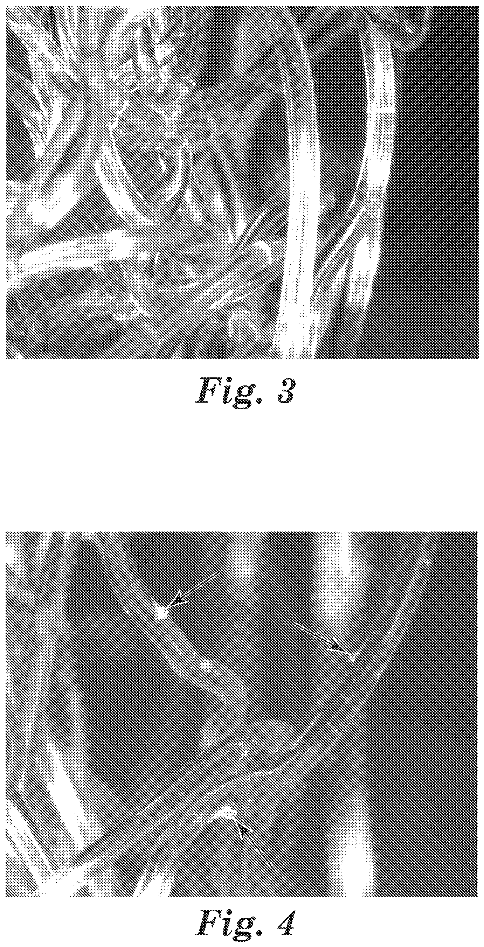

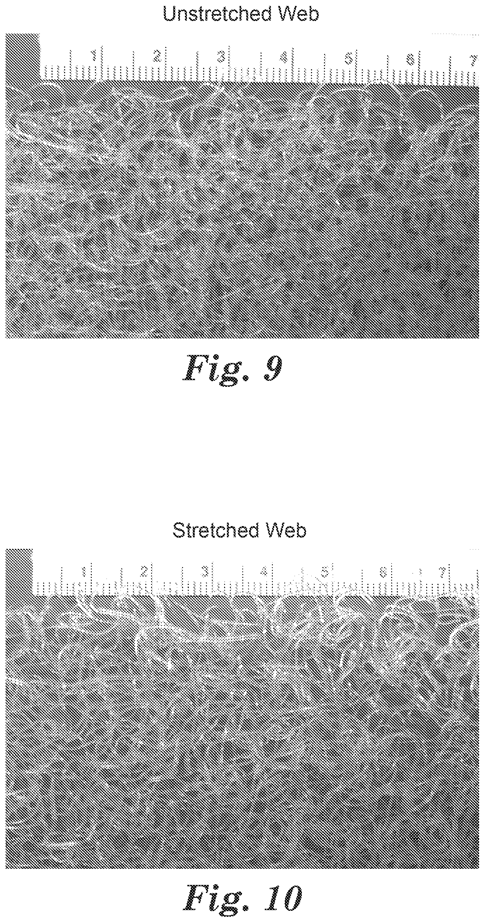

With reference to FIGS. 3 and 9, the coiled-filament web is preferably a three dimensional network of random large diameter thermoplastic filaments. The filaments are randomly coiled or the like and generally can be continuous in one direction, i.e. machine direction.

The filaments form an open network of macropores; which macropores are defined by the nonlinear coiled filaments. The coiled-filament web is characterized as having open cells. The cells are random in size and orientation through the length and depth of the web. The cell structures are generally formed of substantially nonlinear filaments that randomly intersect. The filaments are generally nonlinear between their points of contact or bonding. The web generally has a high void volume, e.g., 40 to 99%, preferably 80% to 99%.

The web is typically a substantially planar web wherein the pores or "open spaces" are uniformly distributed throughout the thickness of the web.

The filaments of the coiled-filament webs are preferably melt-bonded, which means that a plurality or aggregation of such filaments as an open, non-woven web, are bonded together at their points of contact or intersection to form a self supporting bonded structure by either heating the filaments sufficiently, to melt or soften a portion of the filaments or by use of the latent heat of the filaments as extruded. The filaments form lofty webs of interengaged, intertwined, interlocked, or entangled filaments. The filaments are generally helical, spiral, looped, coiled, curly, sinuous or otherwise convoluted and extend from one end of the web to an opposite end of the web. The (e.g. rows of) coiled filaments are continuous in a direction, i.e. the machine direction of the fabrication of the web.

In some embodiments, the coiled-filament web, as can be made by the process of FIG. 2, is further processed, such as by stretching the web in a direction substantially parallel to the direction the coiled filaments are continuous, as described in U.S. Patent Application Serial No. 13/308,962, filed December 1, 2011. In other embodiments, the coiled-filament nonwoven web, as can be made by the process of FIG. 2, is not subject to stretching. Such embodiments will be referred to herein as a "stretched web" and "unstretched web". When the phrase "nonwoven web" or "web" is utilized in the absence of the adjective "stretched" or "unstretched", such phrase refers to both stretched and unstretched webs.

The stretched web is typically produced by stretching the web substantially parallel to the direction the coiled filaments are continuous. By substantially parallel it is meant that the web can be stretched exactly parallel or a deviation from parallel. The deviation from parallel is less than 45 degrees and typically less than 30, 20, 10, or 5 degrees to maximize the benefits obtained by stretching the web parallel to the direction the coiled filaments are continuous. The web is generally stretched at least 2, 2.5 or 3 times the length of the initial web length (i.e. 100%, 150%, or 200%). The web is stretched to a distance no greater than the distance to uncoil the filaments, thereby providing substantially linear parallel filaments when stretched. The upper limit is dependent upon the extent of coiling (i.e. the ratio of actual filament length to coiled filament length). In typical embodiments, the web is stretched no greater than 6, 5 or 4 times the initial web length (i.e. 500%, 400%, or 300%).

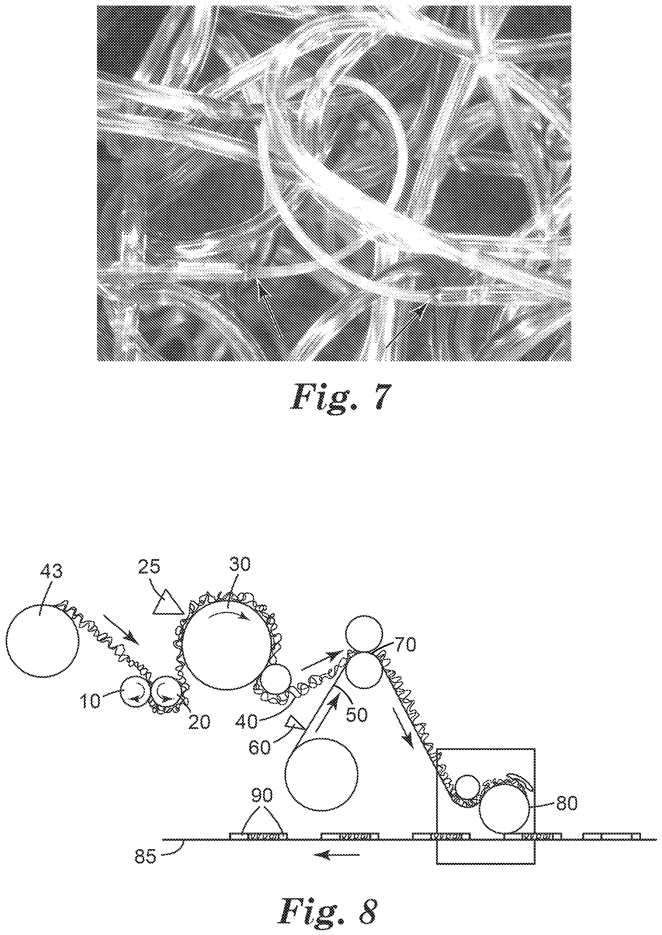

FIG. 1 depicts an illustrative method for producing a stretched and optionally annealed (e.g. coiled-filament) web. In this method, a coiled-filament web 43 (such as prepared by the method depicted in FIG .2) is provided, unwound between a pair of nip rollers 10 and 20 and drawn over a stretching roll 30. The stretching roll surface speed is greater than the speed of the web at the nip, which causes machine direction stretching of the web (at the location between the nip and stretching roll 30). The web is typically further processed to maintain the web in at least a partially stretched configuration. In one embodiment, warmed air 25 is directed at the web while it is held in the stretched state on the stretching roll. The warmed air anneals the stretched thermoplastic filaments of the web, thereby heat setting their stretched configuration. Alternatively, or in combination with annealing, the stretched web can be bonded to a substrate. Such further processing may be conducted during the same process used for stretching the web, such as depicted in FIG. 8. Alternatively, such further processing may be conducted during a subsequent process, such as when converting the web into an assembled intermediate or article. In this embodiment, the stretched web 40 may be wound onto a roll until such further processing.

In some embodiments, the method of producing the web comprises annealing and cooling the stretched web, in order that the stretched configuration of the web is maintained. As depicted in FIG. 1, the stretched web can be annealed and cooled while being maintained in the stretched state. However, for embodiments wherein the stretched web is further fabricated into an assembled intermediate or article such as by bonding the stretched web to another substrate, the annealing step may be optional.

In some embodiments, the stretched web is at least partially relaxed prior to annealing and cooling the stretched web. The relaxed web may be restretched (e.g. about 1-3 times the initial length of the unstretched web). In this embodiment, the restretched web can be annealed and cooled while being maintained in the restretched state.

The stretched web may comprise one or more attributes that are characteristic of being stretched, such as broken bonds, filaments comprising oriented portions, filaments comprising portions of reduced filament diameter, and combinations thereof.

In some embodiments, the web is stretched at a temperature below the softening point of the bonds (e.g. melt-bonds). In doing so, the stretching of the web breaks a portion of the intermittent bonds present between continuous filaments (e.g. in cross-web direction). Thus, the stretched web comprises broken bonds. However, such stretching typically breaks few or no filaments in the machine direction. Hence, the filaments remain continuous during and after stretching, thus maintaining sufficient mechanical integrity of the web.

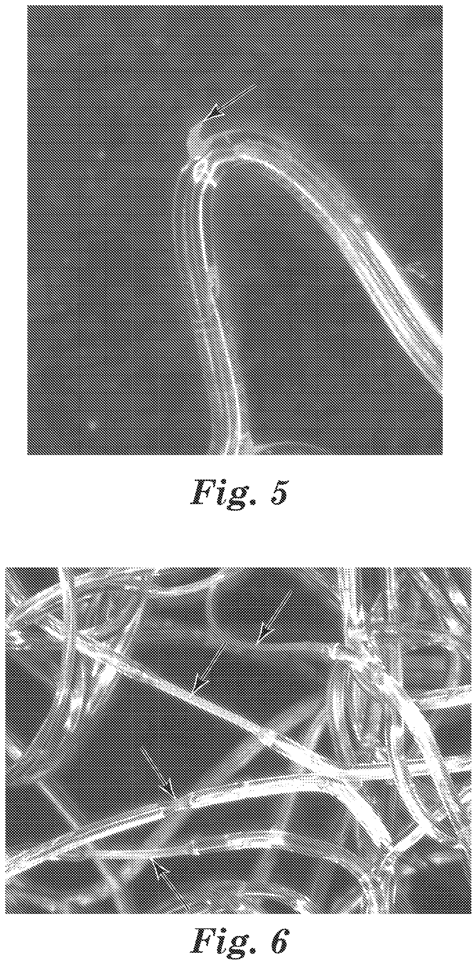

The stretched coiled-filament webs typically exhibit microscopic evidence that prior filament-to-filament bonding points have been broken. The microscopic evidence includes but is not limited to the presence of barbs, thorns, or other protrusion on the filaments, such as depicted in FIGS. 4 and 5. The extent of broken bonds can vary. Although, it is difficult to quantify the total number of broken bonds throughout the thickness of the web, a portion of the broken bonds are readily evident upon microscopy inspection of a single face of the stretched web. In some embodiments, the stretched web may comprise at least 1, 2, or 3 broken bonds per 1 cm² area. Typically, the distribution of the broken bonds is uniformly distributed throughout a single face or the entirety of the stretched web.

The number of broken bonds per area of the stretched web is typically substantial enough such that one or more properties of the web have been altered relative to the unstretched web. For example, the filaments of the unstretched web are typically sufficiently self-bonded such that the web does not substantially elongate in any direction (e.g. machine direction or cross-web direction) without breaking the intermittent bonds between coiled filaments of the web. In contrast, since a substantial portion of the intermittent bonds of the stretched web typically have already been broken, the stretched web can be elongated at least 25% in a direction substantially parallel to the direction the filaments are continuous without breaking (additional) bonds. The extent the stretched web can be elongated (a second time) without breaking additional bonds is about equal to the extent the web was (initially) stretched. In some embodiments the stretched web can be elongated at least 50%, 75%, or 100% in a direction substantially parallel to the direction the filaments are continuous without breaking bonds. In other embodiments, the stretched web can be elongated at least 125%, 150%, 175%, or 200% in a direction substantially parallel to the direction the filaments are continuous without breaking bonds.

Alternatively, or in addition to the presence of broken bonds, the stretched web may comprise filaments comprising oriented portions and/or filaments comprising portions of reduced filament diameter. When the web is stretched at a temperature greater than the softening point of the filament material, but less than the melting point, oriented filament portions and/or filaments comprising portions of reduced filament diameter may be evident in the absence of broken bonds. Whereas the unstretched web typically comprises filaments of about the same filament diameter, as depicted in FIG. 3, the stretched web may comprise filaments wherein a portion of the filament has a substantially smaller diameter. For example, as depicted in FIGS. 5 and 6, the stretched web may comprise filaments wherein a portion of the filament has a diameter about 25% to 70% less than the diameter of the filament adjacent the (e.g. stretched) filament portion. Since the stretched filaments portions having a smaller diameter typically only occupy a relatively small portion of the totality of filaments, the average filament diameter is typically substantially the same or only slightly less than the average filament diameter of the stretched or unstretched web. In some embodiments, the portions of the filaments having a smaller diameter have the same transluscent appearance as the adjacent portion having a greater filament diameter. In other embodiments, the smaller diameter portions have a higher haze or less transparency due to crystalline phases of the thermoplastic materials of the filament becoming oriented during stretching.

In some embodiments, the stretched web generally has a larger pore size than the unstretched web, due to stretching of the filaments and/or breaking of a portion of the intermittent bonds during stretching, such as evident by comparing FIGS. 9 and 10.

In some embodiments, the stretched and preferably relaxed web has a greater length and/or a greater thickness than the unstretched web. The (cross direction) width of the web is at least 90% of the initial web, i.e. prior to stretching. These embodiments are amenable to providing a web having a lower basis weight and/or lower density. This is particularly advantageous when the desired web density is less than can be uniformly and/or efficiently produced by the process of FIG. 2.

In some embodiments, the basis weight of the coiled-filament web is at least 200 gsm or 300 gsm and typically no greater than 800 gsm. In some embodiments, the web has a basis weight no greater than 400 gsm, 350 gsm or 300 gsm.

In some embodiments, the coiled-filament web has a thickness of at least 3 or 4 mm to about 20 mm. In some embodiments, the web has a thickness is no greater than 15, 14, 13, 12, 11 or 10 mm.

The density of the coiled-filament web is typically at least about 0.04 g/cm³ and no greater than about 0.10 g/cm³. In some embodiments, the stretched web has a density no greater than 0.06 g/cm³, 0.05 g/cm³, 0.04 g/cm³. The web may have a density of at least 0.01 g/cm³ or 0.02 g/cm³.

The coiled-filament web preferably exhibits sufficient flexibility, which can be amenable to increased comfort when such web is utilized as a fluid transport element of an assembled intermediate or (e.g. disposable) absorbent article. One property that is indicative to flexibility is work of compression; i.e. the total area under a stress-strain curve (between 0-90 kPa). Work of compression is an indicator of the energy absorbing properties of a material as calculated according to the formula described in US2008/0001431. The web may exhibit a work of compression of no greater than 20 kJ/m³. In some embodiments, the web exhibits a work of compression of no greater than 15 kJ/m³ or 10 kJ/m³. The work of compression of the web is typically at least 2 or 3 kJ/m³. In some embodiments, the work of compression is no greater than 9, or 8, or 7, or 6 kJ/m³.

Another property that is indicative of flexibility is drapability. As used herein drapability refers to the shortest distance from the bottom face of the web to an adjacent vertical structure as determined by the drapability test method described in the examples. Thus, the shorter the distance the more the web bends downwardly. In some embodiments, the web has a drapability less than 100 mm, 90 mm, 80 mm, 75 mm, 70 mm, 65 mm 60 mm, 55 mm, or 50 mm. In some embodiments, the drapability of the stretched web is less than 45 mm, 40 mm, 35 mm, or 30 mm. In some embodiments, the drapability of the stretched web is less than 45 mm, 40 mm, 35 mm, or 30 mm. The drapability may be zero. In some embodiments, the drapability is at least 3 mm, 5 mm, or 10 mm.

The hysteresis properties of the web can be characterized. In some embodiments, the coiled-filament web exhibits a load of less than 25 or 20 or 15 newtons at 25% or 50% extension. In some embodiments, the web exhibits a load of less than 15, or 14, or 13, or 12, or 11, or 10 newtons at 25% extension. In yet other embodiments, the web exhibits a load of less than 9, or 8, or 7, or 6, or 5 newtons at 25% extension. In some embodiments, the web exhibits a load of less than about 20, 15, or 10 newtons at 50% extension.

The webs are typically non-wicking. For example, the nonwoven web may exhibit a vertical wicking of saline solution of no greater than 5, 4, 3, 2, or 1 mm. Such vertical wicking is a property of the web alone, in the absence of being proximate an absorbent material. Without intending to be bound by theory, it is surmised that the web is non-wicking due to the filaments being spaced too far apart to create capillary action. The non-wicking webs exemplified herein are generally prepared from a hydrophobic polymer such as a polyolefin. Further, such non-wicking webs are free of hydrophilic (e.g. cellulose) fibers and superabsorbent polymer.

The coiled-filaments webs were found to provide fast fluid transport rates (as tested accordance to the test method described in the examples). Such fluid transport rate is also a property of the web alone, in the absence of being proximate an absorbent material. In some embodiments, the fluid transport rate was no greater than 10, 9, 8, 7, 6, 5, 4, or about 3 seconds with a weight of 4 kg. In some embodiments, the fluid transport rate was no greater than 15, 14, 13, 12, 11, 10, 9, 8, 7, 6, or about 5 seconds with a weight of 12 kg. In some embodiments, the fluid transport rate was no greater than 20, 19, 18, 17, 16, 15, 14, 13, 12, 11, 10, 9, 8, or about 7 seconds with a weight of 24 kg.

The coiled-filament webs described herein are suitable for use as a fluid transport component of an assembled intermediate, such as suitable for use in a (e.g. disposable) absorbent personal hygiene article. Whereas a finished personal hygiene article typically comprises a fluid transport nonwoven web proximate an absorbent (e.g. core) material between a fluid pervious topsheet and fluid impervious backsheet, an assembled intermediate article lacks at least one requisite component of a finished absorbent article. For example, the assembled intermediate typically lacks a fluid impervious backsheet and/or a fluid pervious topsheet. Thus, the assembled intermediate may be a component of a finished personal hygiene article or other articles.

The assembled intermediate comprises the (e.g. stretched or unstretched) coiled-filament web, as described herein, in combination with at least one other substrate. In some embodiments, the assembled intermediate comprises the coiled-filament web proximate, but not bonded to another substrate. For example, the coiled-filament web may be placed proximate an absorbent material forming an assembled intermediate during the manufacture of an absorbent article. In other embodiments, the assembled intermediate comprises the coiled-filament web bonded to another substrate. For example, the coiled-filament web may be bonded to a (e.g. liquid pervious) carrier substrate, such as a nonwoven or tissue, to facilitate handling of the web by conventional high speed manufacturing processing. In yet another embodiment, the coiled-filament web alone or in combination with a proximate absorbent material may be coated with a (e.g. pressure sensitive) adhesive on at least one major surface that is covered by a release liner carrier substrate. During manufacture of an absorbent article, the release liner is removed and the adhesive is contacted with another component of an absorbent article, such as a liquid impervious backsheet. In yet another embodiment, the coiled-filament web in combination with a proximate absorbent material may be bonded to a carrier substrate such as a nonwoven or film that may subsequently be cut into pieces for and incorporation into an absorbent article.

FIG. 8 depicts one illustrative method of a coiled-filament nonwovens webbeing fabricated into an assembled intermediate. With reference to FIG. 8, the coiled-filament web 40 may be conveyed to an apparatus 80 that cuts the web into discrete pieces and places them proximate an absorbent material 90. The assembled intermediate of the coiled-filament web proximate the absorbent material may be conveyed on a moving belt 85 that conveys the assembled intermediate to subsequent manufacturing operation. Alternatively, the assembled intermediate may be temporarily or permanently bonded to a carrier substrate provided on the belt. When bonded to a carrier substrate, such intermediate may be wound on a roll, to be utilized as a component of an absorbent article. The coiled-filament web may be bonded to another substrate, such as a liquid pervious substrate 50 (e.g. nonwoven, tissue, or fluid acquisition layer). This can be accomplished for example by applying an adhesive to the substrate 50 with an applicator 60 and laminating the adhesive applied substrate to the coiled-filament web 40 in a laminating nip 70, as further depicted in FIG. 8. Alternatively or in combination with a liquid pervious substrate (e.g. nonwoven, tissue, or fluid acquisition layer) being applied to (the upper, topsheet-facing) major face of the web; a substrate may be bonded to the opposing (lower, backsheet-facing) major surface of the web. The inclusion of a substrate, such as tissue, can aid in handling the web in subsequent operation. The inclusion of a substrate, such as tissue, can also aid in the web being cut into discrete pieces for inclusion into the finished composite absorbent constructions using a vacuum wheel type applicator, which is a common and known way of cutting and placing discrete pieces of material onto another material. In the absence of the tissue or other layer to reduce the porosity and air flow of the web, it may be difficult to handle the web using vacuum assisted cutting and placing operations. Although FIG. 8 depicts stretching the web (as shown in FIG. 1), when an unstretched web is utilized, stretching roll 30 and warm air 25 are not needed.

When being bonded to another substrate, the coiled-filament web or more typically the other substrate can be coated with an adhesive or binder on all or a portion of its surface area. Examples of suitable adhesives or binders include emulsion, hot melt, curable, or solvent-based or pressure sensitive adhesives including (meth)acrylate-based pressure sensitive adhesives, such as those described in U.S. Patent No. Re 24,906 (Ulrich), polyurethane adhesives, natural or synthetic rubber-based adhesives, epoxy adhesives, curable adhesives, phenolic adhesives, and the like.

The absorbent (core) material is typically a highly absorbent material that comprises superabsorbent polymer. The absorbent material typically comprises a blend of cellulosic fibers and superabsorbent material. One illustrative absorbent material has a basis weight from about 100 g/m² to about 700 g/m² which has been air-laid as a bottom layer of pulp, a middle layer of pulp and superabsorbent polymer disposed in amongst the pulp, and a top layer containing at least some pulp. The absorbent material may have a density of 0.25 or 0.3 g/cc to about 0.4 g/cc.

The absorbent material typically comprises at least 5 or 10 wt-% and preferably at least 15, 20, 25 or 30 wt-% of superabsorbent polymer. The superabsorbent polymer is typically no greater than 60 wt-% of the absorbent material and in some embodiments, no greater than 55, 50, 45, or 40 wt-%. The absorbent material may have a basis weight of at least 150 to 200 g/m² and typically no greater than 300 or 350 g/m².

Various absorbent (core) materials and methods of making such have been described in the art. (See for example US 4,610,678 and US 6,896,669)

In some embodiments, the fluid uptake rate (as tested in accordance to the test method described in the examples) was no greater than 10, 9, 8, 7, 6, 5, or about 4 seconds with a weight of 4 kg. Notably commercially available products that were tested were found to have a fluid uptake rate of about 23-31 seconds with a weight of 4 kg. In some embodiments, the fluid uptake rate was no greater than 50, 40, 30, 20, and in some embodiments no greater than 15seconds with a weight of 12 kg. Notably commercially available products that were tested were found to have a fluid uptake rate of about 98-102 seconds with a weight of 12 kg. In some embodiments, the fluid uptake rate was no greater than 50 or 40 seconds, and in some embodiments no greater than 35 or about 30 seconds with a weight of 24 kg.

Notably commercially available products that were tested were found to have a fluid uptake rate of about 102-182 seconds with a weight of 24 kg. Such fluid uptake performance can be achieved with a single fluid challenge (100 ml of 0.9% NaCl in water) or at least two (2 - 100 ml doses of 0.9% NaCl in water separated by a time interval of 2 minutes). Although the fluid uptake rate was measured on a finished personal hygiene article, the topsheet and backsheet are surmised to have little or no effect on the test results. Hence, the fluid uptake rate is surmised a property of an assembled intermediate of the coiled-filament web proximate an absorbent material.

Another property that is indicative of the fluid transport properties of the coiled-filament web is length of longitudinal fluid distribution (as tested in accordance to the test method described in the examples). In some embodiments, the length of longitudinal fluid distribution is at least 100, 110, 120, 130, 140 mm, and in some embodiments at least 150 or 160 mm with a weight of 4 kg. Notably commercially available products that were tested were found to have a length of longitudinal fluid distribution of 85 mm. In some embodiments, the length of longitudinal fluid distribution is at least 100, 125, 150, 160 mm, and in some embodiments at least 170, 180, 190, or 200 mm with a weight of 12 kg. Notably commercially available products that were tested were found to have a length of longitudinal fluid distribution of 78-85 mm. In some embodiments, the length of longitudinal fluid distribution is at least 125, 150, 160 mm, and is some embodiments at least 170 mm with a weight of 24 kg. Notably commercially available products that were tested were found to have a length of longitudinal fluid distribution of 95-100 mm.

The coiled-filament web, utilized as a fluid transport element of an absorbent component can have various shapes including symmetrical (having a point, line, or plane of symmetry) or unsymmetrical shapes. Coiled web shapes that are envisioned include but are not limited to circles, ovals, squares, rectangles, pentagons, hexagons, octagons, trapezoids, truncated pyramids, hourglasses, dumbbells, dog bones, etc. The edges and corners can be straight or rounded. The sides can be curved (convex or concave), tapered, flared, or angled. In addition, the coiled web can contain cut-out regions that create voids, cavities, depressions, channels, or grooves. In some embodiments, the shape of the coiled web is preferably rectangular. Regardless of the shape, a coiled-filament web fluid transport elements can generally be defined as having a first major face, an opposing second major face substantially parallel to the first major face, and a thickness in a direction orthogonal to the first and opposing major face. The coiled-filament web may comprise various functional additives including for example, antimicrobial coatings, ion capturing coatings, desiccants, and odor control particles.

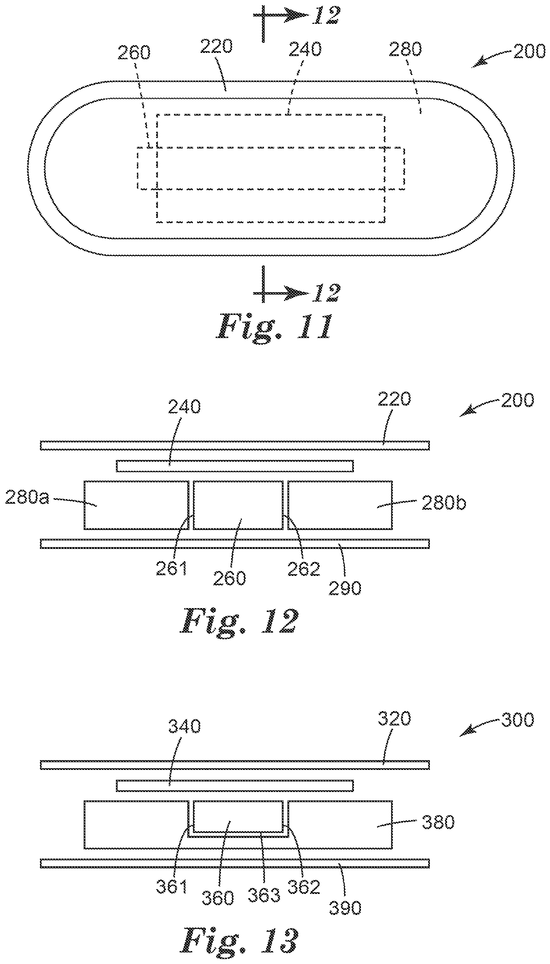

There are various ways in which the coiled-filament web can be utilized as a fluid transport element proximate an absorbent material, some of which are depicted in FIGS. 11-19, as will subsequently be described. In some favored embodiments, the thickness of the coiled-filament web defines lateral edges that are at least partially in fluid communication with the absorbent material. In some embodiments, the lateral edges of the coiled-filament web are in fluid communication with the absorbent material by direct contact. In other embodiments, a low absorbency layer (i.e. lacking superabsorbent polymer), such as a tissue layer, may be present between the lateral edges of the coiled-filament web and the absorbent material.

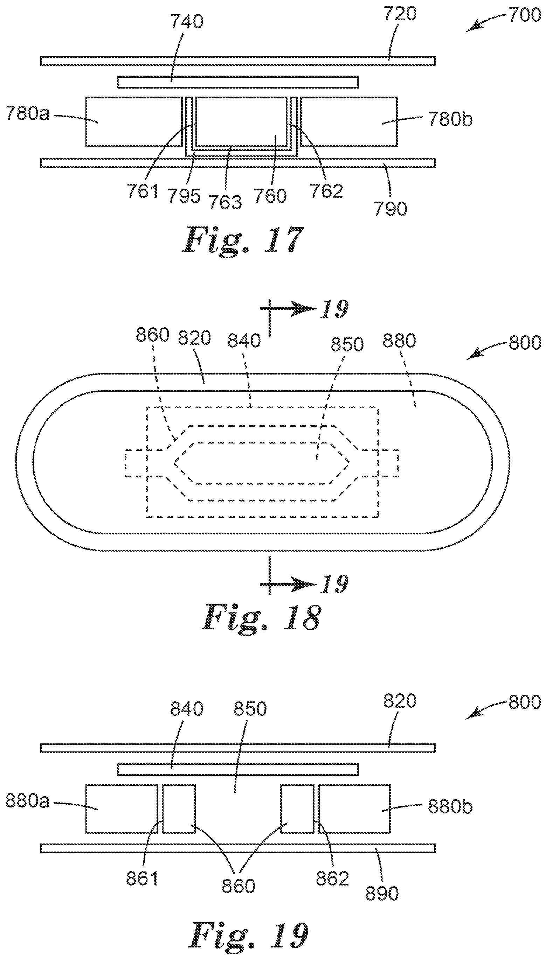

In some favored embodiments, the coiled-filament web is inserted within an absorbent material such that substantially all the lateral edges are in fluid communication with the absorbent material. This arrangement can have faster fluid uptake. However, the length of longitudinal fluid distribution may be less. In yet other embodiments, at least two opposing lateral edges of the nonwoven web are in contact with the absorbent material. For example the lateral edges at the end-portion of the coiled-filament web may be in contact with the absorbent material. If the coiled-web is rectangular shaped, the end portions correspond to the lateral peripheral edges that define the width and typically a portion of the length. However, two opposing lateral peripheral edges along the length of the central-portion of the coiled-filament web may be rendered fluid impervious for example by sealing the edge or by including a fluid impervious film between such lateral edges and the adjacent absorbent material, as will further be explained with reference to FIG. 17. This arrangement can have slower fluid uptake. However, the length of longitudinal fluid distribution may be greater.

The assembled intermediate comprising the coiled-filament web (fluid transport element) proximate an absorbent material may further comprise another substrate prior to incorporation into a (disposable) absorbent article. Common substrates that are often included in a (disposable) absorbent article include topsheets, acquisition distribution layers (ADLs), and backsheets.

The topsheet is typically compliant, soft feeling, and non-irritating to the wearer's skin. Further, the topsheet is liquid pervious, permitting liquids to readily penetrate through its thickness. Suitable topsheets may be manufactured from a wide range of materials, such as porous foams, reticulated foams, apertured plastic films, natural fibers (e.g., wood or cotton fibers), synthetic fibers (e.g., polyester or polypropylene fibers) or from a combination of natural and synthetic fibers. The topsheet is typically a hydrophobic material to isolate the wearer's skin from liquids in the absorbent material.

There are a number of manufacturing techniques which may be used to manufacture the topsheet. The topsheet may be woven, non-woven, spunbonded, carded, or the like. An illustrative topsheet is carded, and thermally bonded (1.5 denier polypropylene stable fibers). The topsheet may have a basis weight from about 18 to about 25 grams per square meter. Further, the topsheet typically has a minimum dry tensile strength of at least about 400 grams per centimeter in the machine direction and a wet tensile strength of at least about 55 grams per centimeter in the cross machine direction. An acquisition distribution layer (also sometimes called a "surge" layer) is typically disposed beneath the topsheet and over the absorbent core. Acquisition layers have typically been constructed of a woven, non-woven or carded fibrous material. They are arranged to quickly absorb the liquid through the absorbent article's top sheet for temporary retention (e.g., to act as a temporary reservoir), and to transfer that liquid into the underlying absrobent core at a rate at which the core can absorb for final or permanent retention. The acquisition layer typically improves "wicking" of the absorbent article by spreading the body fluid in the "x" and "y" plane over the area of the core encompassed by the acquisition layer while also carrying the fluid in the "z" direction to the absorbent core. Examples of commercially available materials used for acquisition layers in disposable absorbent articles are through-air bond staple fibers, adhesively bonded staple fibers, and thermally point bonded staple fibers.

The backsheet is impervious to liquids and typically is a thin plastic film, although other liquid impervious materials may also be used. The backsheet is typically flexible, meaning that it is compliant and will readily conform to the general shape and contours of the wearer's body. The backsheet prevents the exudates absorbed and contained in the absorbent material from wetting articles which contact the absorbent article such as bed sheets and undergarments. One illustrative backsheet is polyethylene film having a thickness of from about 0.012 mm (0.5 mil) to about 0.051 centimeters (2.0 mils). The backsheet may be embossed and or matte finished to provide a more clothlike appearance. Further, the backsheet may permit vapors to escape from the absorbent member while still preventing exudates from passing through the backsheet.

In a typical disposable absorbent article, the topsheet and backsheet are associated together in any suitable manner. Typically, the topsheet and the backsheet are affixed directly to each other at the periphery of the article by an attachment means such as an adhesive or any other attachment means as known in the art.

FIG. 11 depicts a top view of an illustrative absorbent article 200 comprising an assembled intermediate of a coiled-filament web fluid transport element 260 proximate an absorbent (e.g. core) material 280. Absorbent article 200 further comprises a fluid pervious topsheet 220, a fluid pervious acquisition distribution layer (ADL) 240 beneath and in fluid communication with the top sheet 220, and a coiled-filament web fluid transport element 260 beneath and in fluid communication with the ADL. The absorbent (e.g. core) material 280 typically surrounds the fluid transport element 260.

FIG. 12 is a central cross-sectional view of the illustrative assembled intermediate and absorbent article of FIG. 11. Coiled-filament web fluid transport element 260 comprises lateral edge 261 in fluid communication, such as by direct contact with absorbent (core) portion 280a and lateral edge 262 in fluid communication, such as by direct contact with absorbent (core) portion 280b.

FIGS. 13-16 and FIG. 19 depict cross-sectionals views of other illustrative assembled intermediates, comprising a fluid transport element (360, 460, 560, 660, and 860) proximate an absorbent material (380, 480, 580, 680 and 880), incorporated into an absorbent article. In each of the cross-sectional views of FIGS. 13-16 and FIG. 19, the thickness of the coiled-filament web fluid transport element defines lateral edges (361,362; 461, 462; 561, 562; 661, 662; 861,862) and the lateral edges are in fluid communication, such as by being in direct contact with the absorbent material.

In the illustrative embodiments of FIG. 12, FIGS. 14-16 and FIG. 19, the opposing major face of the coiled-filament web fluid transport element (260, 460, 560, 660, and 860) is in direct contact with fluid impervious backsheet element (290, 490, 590, 690, and 890). A tissue layer (not shown) may optionally be present between the fluid impervious backsheet element (290, 490, 590, 690, and 890) and coiled-filament web fluid transport element (260, 460, 560, 660, and 860). Such tissue can be attached directly to the fluid transport element to aid in handling. In the embodiments depicted in FIG. 12, FIGS. 14-16 and FIG. 19; a highly absorbent (core) material (i.e. comprising super absorbent polymer) is not present between the fluid impervious backsheet element (290, 490, 590, 690, 890) and coiled-filament web fluid transport element (260, 460, 560, 660, and 860).

FIG. 13 is a central cross-sectional view of another illustrative assembled intermediate incorporated into absorbent article 300. This embodiment comprises a fluid pervious topsheet 320, a fluid pervious acquisition distribution layer (ADL) 340 beneath and in fluid communication with the top sheet 320, and a coiled-filament web fluid transport element 360 beneath and in fluid communication with the ADL. Coiled-filament web fluid transport element 360 comprises lateral edges 361 and 362 in fluid communication, such as by direct contact with absorbent (core) material 380. Unlike the embodiments depicted in FIG. 12, FIGS. 14-16 and FIG. 19; in this embodiment, opposing (bottom) major face 363 of coiled-filament web fluid transport element 360 is in contact with absorbent core material 380. Thus, a layer of highly absorbent (core) material 380 is present between the liquid impervious backsheet 390 and coiled-filament web fluid transport element 360. In this embodiment, the surface area of the coiled-filament web fluid transport element 360 in fluid communication with absorbent core material 380 can be greater than FIG. 12 for example.

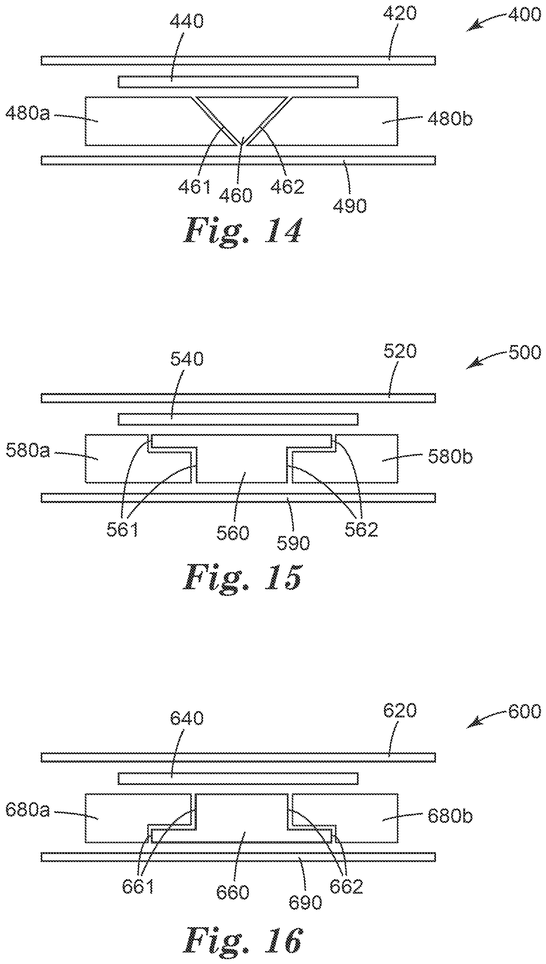

FIG. 14 is a central cross-sectional view of another illustrative assembled intermediate incorporated into absorbent article 400. This embodiment comprises a fluid pervious topsheet 420, a fluid pervious acquisition distribution layer (ADL) 440 beneath and in fluid communication with the top sheet 420, and a coiled-filament web fluid transport element 460 beneath and in fluid communication with the ADL. Coiled-filament web fluid transport element 460 comprises lateral edge 461 in fluid communication with absorbent material portion 480a and lateral edge 462 in fluid communication with absorbent material portion 480b, such as by direct contact with absorbent (core) material 480. In this embodiment, the coiled-filament web fluid transport element 460 has a triangular shaped cross-section with one of the apexes of the triangle in contact with liquid impervious backsheet 490. In this embodiment, the surface area of lateral edges 461 and 462 of coiled-filament web fluid transport element 460 can be greater than the surface area of the lateral edges of a coiled-filament web having a rectangular cross section (such as shown in FIG. 12).

FIG. 15 is a central cross-sectional view of another illustrative assembled intermediate incorporated into absorbent article 500. This embodiment comprises a fluid pervious topsheet 520, a fluid pervious acquisition distribution layer (ADL) 540 beneath and in fluid communication with the top sheet 520, and a coiled-filament web fluid transport element 560 beneath and in fluid communication with the ADL. Coiled-filament web fluid transport element 560 comprises lateral edges 561 and 562 in fluid communication with absorbent material portions 580a and 580b respectively, such as by direct contact with absorbent (core) material 580. In this embodiment, the coiled-filament web fluid transport element 560 has a T-shaped cross-section with the bottom surface in contact with backsheet 590.

FIG. 16 is a central cross-sectional view of another illustrative assembled intermediate incorporated into absorbent article 600. This embodiment comprises a fluid pervious topsheet 620, a fluid pervious acquisition distribution layer (ADL) 640 beneath and in fluid communication with the top sheet 620, and a coiled-filament web fluid transport element 660 beneath and in fluid communication with the ADL. Coiled-filament web fluid transport element 660 comprises lateral edges 661 and 662 in fluid communication with absorbent material portions 680a and 680b respectively, such as by direct contact with absorbent (core) material 680. In this embodiment, the coiled-filament web fluid transport element 660 has a inverted T-shaped cross-section with the bottom surface in contact with backsheet 690.

FIG. 17 is a central cross-sectional view of another illustrative assembled intermediate incorporated into absorbent article 700. This embodiment comprises a fluid pervious topsheet 720, a fluid pervious acquisition distribution layer (ADL) 740 beneath and in fluid communication with the top sheet 720, and a coiled-filament web fluid transport element 760 beneath and in fluid communication with the ADL. Coiled-filament web fluid transport element 760 comprises lateral edges 761 and 762 and bottom edge 763 in contact with a liquid impervious (e.g. film) layer 795 that envelopes the coiled-filament web fluid transport element 760 on both lateral edges and the opposing (bottom) surface. In some embodiments, this liquid impervious (e.g. film) layer has the same length as the fluid transport nonwoven web. In other embodiments, this liquid impervious (e.g. film) layer may have a shorter length and only present at the central region (about 50%) of the article. In this embodiment, the article at the end portions of the fluid transport nonwoven web (in cross-section) may have the same appearance as FIG. 12.

FIG. 18 depicts a top view of another illustrative assembled intermediate incorporated into absorbent article 800 comprising a fluid pervious topsheet 820, a fluid pervious acquisition distribution layer (ADL) 840 beneath and in fluid communication with the top sheet 820, and a coiled-filament web fluid transport element 860 beneath and in fluid communication with the ADL. The absorbent (e.g. core) material 880 typically surrounds the fluid transport element 860.

FIG. 19 is a central cross-sectional view of the assembled intermediate and absorbent article of FIG. 18. As best shown in shown in FIG. 19, this embodiment further comprises a void 850 (an air-space). The coiled-filament web fluid transport element 860 is typically sufficiently resilient such that the air-space is maintained with moderate compression.

In some embodiments, the fluid transport element (e.g. 260 of FIG. 11) has a dimension (e.g. length) that extends beyond the length (i.e. longest dimension) of the ADL. In such embodiment, the fluid transport element may also be in fluid communication such as by direct contact with the topsheet. Alternatively, the dimension of the fluid transport element 260 may be selected such that the first major face of fluid transport element 260 is in contact with the ADL and not with the topsheet.

The assembled intermediates or absorbent articles may further comprise one or more tissue layers between the ADL and fluid transport element. Such tissue layers are typically not "highly absorbent" as in the case of the absorbent (e.g. core) material comprising superabsorbent polymer.

The invention is illustrated by the following non-limiting examples.

### Coiled Web Preparation

### Examples 1-10 (Unstretched Web)

Coiled webs were prepared according to the method described in United States Patent No. 6,762,139 (Strommen). A Haake model extruder (Thermo Electron Corporation, Newington, NH) with a one inch screw was used. The die temperature was set at 260-302 °C. The cooling bath was a 100 gallon water bath maintained at 21-27 °C. TRITON GR-5M surfactant (0.025%, Dow Chemical Company, Midland, MI) was added to the cooling bath. The basis weight of the coiled webs was controlled by individual adjustments to the resin throughput of the extruder and the line speed for withdrawing the web from the cooling bath. The screw speed of the extruder ranged from 5-100 rpm and the line speed for withdrawing the web from the cooling bath ranged from 5-15 meters/minute. The coiled webs of Examples 1-2 were prepared using ELITE-5815 polyethylene thermoplastic resin (Dow Chemical Company, Midland, MI). The coiled webs of Examples 3-6 were prepared using VERSIFY-4200 polypropylene thermoplastic resin (Dow Chemical Company, Midland, MI). The coiled web of Example 7 was prepared from a 1:1 weight ratio blend of ELITE-5815 and ENGAGE-8407 polyethylene resin (Dow Chemical Company, Midland, MI). The coiled webs of Examples 8-9 were prepared using DOW C700-35N polypropylene impact copolymer thermoplastic resin (Dow Chemical Company, Midland, MI). The coiled web of Example 10 was prepared using nylon 6 resin (commercially available as ULTRAMID Polyamide 6 from the BASF Corporation, Wyandotte, MI). For Examples 1-9, a 102 mm by 19 mm patterned extrusion die was used that contained 117 (760 micron in diameter) holes evenly spaced in five rows. The width of the coiled webs was 10 cm as measured in the cross direction (CD). The measured values of filament diameter (microns), web basis weight (gsm), web thickness, and web density are reported in Table 1 for Examples 1-10.

### Examples 11-21 (Stretched Web)

The coiled-filament stretched webs of Examples 11-21 were prepared by providing an unstretched precursor web selected from Examples 1-10 and further processing of the precusor webs by a combination of hand stretching and annealing. A sample of coiled web measuring 30 cm in the machine direction (MD) and 10 cm in the cross direction (CD) was used. Two ink marks were made with each mark positioned 10 cm from the edge in the MD. The sample was grasped by hand at the marked positions and stretched in the MD until the two marks were either 30 or 40 cm apart (a 3:1 or 4:1 stretch ratio). During this hand stretching operation, numerous filament to filament bonds in the coiled web were broken. The tension was removed and the web was allowed to return to a relaxed state. In the relaxed state, the stretched portion of the web was significantly less than 40 cm, with lengths between the two ink marks measuring about 12-13 cm. The stretched section of the web was hand stretched a second time to a position where the ink marks were held 20-25 cm apart (approximate 2:1 stretch ratio). The sample was held in the stretched position, clamped to a piece of cardboard, and placed in a hot air oven set at 65 °C for 30-45 seconds. The stretched and annealed sample was removed from the oven, maintained at ambient temperature for 1 minute, and then released from the clamping apparatus to provide the final product.

The hand stretching method described above was used to prepare the stretched coiled webs of Examples 11-21. Examples 11-13 were prepared starting with Example 1, while Examples 14-16 were prepared starting with Example 7 and Examples 17-21 were prepared starting with Example 3. The starting coiled web was 6.2 mm thick for Example 1, 7.1 mm thick for Example 7, and 7.2 mm thick for Example 3. In Table 2, measured values are reported for the distance of the initial web stretch, the relaxed distance of stretched web material after the initial stretch, the distance of the second web stretch, and the relaxed distance of the stretched web material after the second stretch and annealing step. For Examples 11-21, data was collected from three replicates and the mean values are reported.

In Table 3, the measured values of web basis weight (gsm), web thickness (mm), and web density (g/cm³) are reported for Examples 11-21. For each of the three web properties, the difference between the value measured for the stretched web and the value measured for the unstretched precursor web was calculated. A calculation of the percent increase or decrease in the value was also done. The calculations are reported in Table 4 and show that stretching the coiled webs resulted in the formation of webs with increased thickness, decreased basis weight and decreased density.

**Table 1. Unstretched Coiled Webs**

| | Filament Diameter (microns) | Web Basis Weight (gsm) | Web Thickness (mm) | Web Density (g/cm³) |
|---|---|---|---|---|
| Example 1 | 357.6 | 541.3 | 6.2 | 0.087 |
| Example 2 | 400.3 | 366.7 | 4.7 | 0.078 |
| Example 3 | 374.7 | 480.8 | 7.2 | 0.067 |
| Example 4 | 495.4 | 561.8 | 12.9 | 0.043 |
| Example 5 | 701.9 | 705.8 | 11.4 | 0.062 |
| Example 6 | 736.1 | 531.7 | 7.9 | 0.067 |
| Example 7 | 347.7 | 422.4 | 7.1 | 0.059 |
| Example 8 | 501.5 | 514.9 | 9.5 | 0.054 |
| Example 9 | 496.0 | 473.6 | 5.6 | 0.084 |
| Example 10 | 292.5 | 400.5 | 12.6 | 0.032 |

**Table 2. Data from the Process for Hand Stretching Coiled Webs**

| Example No. | Distance of First Web Stretch (cm) | Relaxed Distance of Web After First Stretch (cm) | Distance of Second Web Stretch (cm) | Relaxed Distance of Web After Second Stretch and Annealing (cm) |
|---|---|---|---|---|
| Example 11 | 40 | 12.8 | 25 | 22.3 |
| Example 12 | 30 | 12.5 | 22 | 19.8 |
| Example 13 | 40 | 12.7 | 20 | 18.0 |
| Example 14 | 40 | 12.7 | 25 | 23.7 |
| Example 15 | 30 | 12.2 | 22 | 20.5 |
| Example 16 | 40 | 12.7 | 20 | 19.8 |
| Example 17 | 40 | 11.8 | 25 | 21.0 |
| Example 18 | 30 | 11.0 | 22 | 18.7 |
| Example 19 | 40 | 12.2 | 20 | 17.7 |
| Example 20 | 30 | 11.5 | not done | not done |
| Example 21 | 40 | 12.5 | not done | not done |

**Table 3. Stretched Coiled Webs**

| | Web Basis Weight (gsm) | Web Thickness (mm) | Web Density (g/cm³) |
|---|---|---|---|
| Example 11 | 294.0 | 7.8 | 0.038 |
| Example 12 | 226.0 | 9.4 | 0.024 |
| Example 13 | 287.8 | 10.0 | 0.029 |
| Example 14 | 222.7 | 8.6 | 0.026 |
| Example 15 | 267.0 | 8.1 | 0.033 |
| Example 16 | 255.3 | 10.0 | 0.025 |
| Example 17 | 257.7 | 8.5 | 0.030 |
| Example 18 | 304.8 | 8.5 | 0.035 |
| Example 19 | 305.9 | 9.1 | 0.034 |
| Example 20 | 463.8 | 8.3 | 0.056 |
| Example 21 | 417.8 | 9.1 | 0.046 |

**Table 4. Calculations for Stretched Coiled Webs**

| | Decrease in Web Basis Weight (gsm) | Percent Decrease in Web Basis Weight | Increase in Web Thickness (mm) | Percent Increase in Web Thickness | Decrease in Web Density (g/cm³) | Percent Decrease in Web Density |
|---|---|---|---|---|---|---|
| Example 11 | 247.3 | 46% | 1.6 | 26% | 0.049 | 56% |
| Example 12 | 315.3 | 58% | 3.2 | 52% | 0.063 | 72% |
| Example 13 | 253.5 | 47% | 3.8 | 61% | 0.058 | 67% |
| Example 14 | 199.7 | 47% | 1.5 | 21% | 0.033 | 56% |
| Example 15 | 155.4 | 37% | 1.0 | 14% | 0.026 | 44% |
| Example 16 | 167.1 | 40% | 2.9 | 41% | 0.034 | 58% |
| Example 17 | 223.1 | 46% | 1.3 | 18% | 0.037 | 55% |
| Example 18 | 176.0 | 37% | 1.3 | 18% | 0.032 | 48% |
| Example 19 | 174.9 | 36% | 1.9 | 26% | 0.033 | 49% |
| Example 20 | 17.0 | 4% | 1.1 | 15% | 0.011 | 16% |
| Example 21 | 63.0 | 13% | 1.9 | 26% | 0.021 | 31% |

### Photomicrograph Examination of Webs

Photomicrographs of unstretched coiled webs (Examples 1 and 7) and stretched coiled webs (Examples 13 and 15) were taken using a Leica Model MZ16 stereomicroscope at a magnification of 7.1X (commercially available from Leica Microsystems, Wetzlar, Germany). The photomicrographs were examined for broken filament to filament bonds in the webs. The web sample was laid flat on the stage of the microscope and photomicrographs were taken from the top face of the web in five randomly selected regions of the web. All of the photomicrographs were of samples measuring 0.83 cm² in size (10.5 mm (MD) by 7.9 mm (CD)). Two individuals examined the photomicrographs and independently counted barbs. This resulted in a total of ten replicates for each sample. The individual values were averaged and the mean value for each coiled web example was determined. The results are reported in Table 5. In the test method, examination of the photomicrographs was limited to counting barbs that projected sideways from a filament. Barbs hidden behind a filament or projecting into or away from the camera were not clearly visible and consequently not counted. The actual number of barbs in the sample area was greater than the number determined by the test method.

**Table 5**

| | Type of Web | Mean Number of Broken Bonds (n=10) |
|---|---|---|
| Example 1 | unstretched | 0 |
| Example 13 | stretched | 3.9 |
| Example 7 | unstretched | 0.2 |
| Example 15 | stretched | 3.8 |

### Compressibility

The energy required to compress the coiled webs was measured. All testing was conducted at constant temperature (23°C ± 2°C) and relative humidity (50% ± 5%). All materials and equipment were equilibrated at these conditions for a minimum of 24 hours prior to testing. A universal constant rate of extension tensile testing instrument equipped with a computer for data recording and the required load ranges was used (Series 4200, 4500, or 5500 available from Instron Engineering Corporation, Canton, MA). The instrument crosshead speed was set to 200 mm/minute and the calibrated load cell used was rated for 500 N. The finished sample of coiled web was cut in a 3 inch diameter circle and the thickness was measured using a digital hand-held caliper. Three replicates of fresh materials were used for each sample and the data is reported as the average of the recorded values.

The Instron instrument was fitted with two compression platens (6 inch diameter) aligned in parallel with one attached to the lower jaw creating the base and one attached to the upper jaw creating the moving piston that applied the compressive force. The platens were brought into contact and the jaw gap measurement was set to zero on the instrument. The platens were then drawn apart to a distance equal to the sample thickness. The gauge length was reset to zero and then manually jogged a nominal distance further apart to allow for sample placement. The sample was placed on the lower platen and the top platen was returned to the zero position against the sample. The sample was then compressed with automatic recording of the compressibility (percent strain) at compressive stresses (kPa) of 1, 5, 10, 20, 40, 60, and 100 kPa. The area under the plotted stress-strain curve (between 0-90 kPa) was calculated and reported as the Work of Compression (WOC) in kilojoules/m³ (Tables 6 and 7).

The difference between the WOC value measured for each stretched web sample (Examples 11-13, 15, and 17-21) and the WOC measured for the corresponding unstretched precursor web was calculated. The percent decrease in WOC was also determined. The results are reported in Table 8. The data shows that stretching a coiled web resulted in a decrease in the WOC measurement.

**Table 6. Compressibility Data for Unstretched Coiled Webs**

| | Percent Strain (%) | | | | | | | |
|---|---|---|---|---|---|---|---|---|
| Example | at 1 kPa | at 5 kPa | at 10 kPa | at 20 kPa | at 40 kPa | at 60 kPa | At 100 kPa | WOC (kJ/m³) |
| Example 1 | 13.9 | 30.7 | 45.8 | 59.3 | 68.5 | 73.0 | 78.6 | 10.2 |
| Example 2 | 14.5 | 24.9 | 35.8 | 45.9 | 52.0 | 55.9 | 60.3 | 7.4 |
| Example 3 | 14.9 | 42.6 | 61.7 | 74.9 | 82.9 | 86.5 | 91.0 | 9.6 |
| Example 4 | 39.6 | 60.3 | 73.3 | 81.7 | 87.0 | 89.2 | 92.1 | 6.6 |
| Example 5 | 28.3 | 35.4 | 45.1 | 61.0 | 74.1 | 79.3 | 84.7 | 11.6 |
| Example 6 | 18.6 | 28.1 | 37.4 | 50.6 | 61.2 | 65.6 | 70.8 | 10.0 |
| Example 7 | 13.3 | 54.8 | 66.7 | 73.4 | 78.5 | 81.3 | 85.1 | 7.6 |
| Example 8 | 0.0 | 8.7 | 17.0 | 33.9 | 52.1 | 61.7 | 70.5 | 29.3 |
| Example 9 | 0.0 | 0.0 | 9.4 | 17.7 | 25.1 | 33.7 | 45.1 | 30.5 |
| Example 10 | 3.9 | 25.6 | 42.9 | 62.4 | 75.4 | 80.5 | 85.2 | 19.2 |

**Table 7. Compressibility Data for Stretched Coiled Webs**

| | Percent Strain (%) | | | | | | | |
|---|---|---|---|---|---|---|---|---|
| Example | at 1 kPa | at 5 kPa | at 10 kPa | at 20 kPa | at 40 kPa | at 60 kPa | at 100 kPa | WOC (kJ/m³) |
| Example 11 | 20.3 | 45.8 | 54.2 | 59.2 | 63.5 | 65.6 | 68.8 | 5.9 |
| Example 12 | 36.3 | 60.7 | 68.6 | 75.2 | 79.0 | 81.1 | 83.8 | 8.1 |
| Example 13 | 38.3 | 64.9 | 72.9 | 79.0 | 83.1 | 85.2 | 88.1 | 5.6 |
| Example 15 | 52.6 | 71.9 | 75.8 | 80.5 | 82.7 | 84.3 | 86.2 | 3.7 |
| Example 17 | 35.3 | 65.4 | 72.8 | 78.6 | 81.9 | 83.6 | 86.1 | 5.1 |
| Example 18 | 26.4 | 58.6 | 68.1 | 74.8 | 78.9 | 81.0 | 83.8 | 6.1 |
| Example 19 | 25.7 | 54.0 | 63.4 | 70.5 | 74.6 | 76.8 | 79.5 | 5.9 |
| Example 20 | 13.6 | 40.9 | 54.8 | 65.9 | 72.2 | 75.3 | 79.2 | 8.2 |
| Example 21 | 19.6 | 47.5 | 58.2 | 66.5 | 71.0 | 73.4 | 76.4 | 6.5 |

**Table 8. Decrease in WOC for Stretched Coiled Webs**

| Example | Decrease in WOC (kJ/m³) | Percent Decrease in WOC |
|---|---|---|
| Example 11 | 4.3 | 42% |
| Example 12 | 2.1 | 21% |
| Example 13 | 4.6 | 45% |
| Example 15 | 3.9 | 51% |
| Example 17 | 4.5 | 47% |
| Example 18 | 3.5 | 36% |
| Example 19 | 3.7 | 39% |
| Example 20 | 1.4 | 15% |
| Example 21 | 3.1 | 32% |

### Vertical Wicking

A shallow aluminum tray was filled with saline solution (0.9% NaCl in water) to a depth of 12.7 mm. To enhance visualization, the saline solution was dyed with red food coloring. Test samples of Examples 3 and 17 and Comparative Examples A1-A3 were prepared as 25.4 mm by 152.4 mm strips. Comparative Example A1 was the acquisition distribution layer (ADL) obtained from a size six baby diaper commercially available from the Kimberly Clark Corporation, Neenah, WI, under the trade designation "HUGGIES LITTLE MOVERS". The ADL was a 3 mm thick nonwoven web having an average basis weight of 106 gsm and an average fiber diameter of 30 microns. Comparative Example A2 was the absorbent core obtained from an adult incontinence pad commercially available from the SCA Personal Products, Philadelphia, PA, under the trade designation "TENA SERENITY". The absorbent core was a bonded, air-laid material containing cellulosic fibers and superabsorbent polymer layered between two sheets of tissue. The absorbent core was 5 mm thick and had an average basis weight of 440 gsm. Comparative Example A3 was a "WYPALL L30" nonwoven tissue commercially available from the Kimberly Clark Corporation. A clip was attached to the narrow end of each sample and the samples were individually hung from a support located above the tray. The samples were oriented so that they were positioned perpendicular to the tray and were submerged into the saline solution so that the free ends of the samples touched the bottom of the tray. The samples were maintained in the saline solution for 60 minutes at ambient temperature. The samples were removed from the saline solution and the distance of travel by the saline solution in each sample was measured with a ruler (Table 9). The samples were subsequently drip dried by suspending in air for five minutes and then weighed. The percent weight gain from the fluid held by the web is reported in Table 9.

**Table 9**

| | Distance of Travel by the Saline Solution (mm) | Percent Weight Gain |
|---|---|---|
| Example 3 (unstretched) | 0.0 | 1.4% |
| Example 17 (stretched) | 0.0 | 5.9% |
| Comparative Example A1 | 9.5 | 120.8% |
| Comparative Example A2 | 76.2 | 912.2% |
| Comparative Example A3 | 127 | 450.0% |

### Drapability

A rectangular cube with solid horizontal and vertical surfaces was used as the test structure for drapability measurements. A 101.6 mm (CD) by 152.4 mm (MD) sample of the nonwoven web was laid flat on the top surface of the cube and positioned so that 76.4 mm (in MD direction) of the sample hung over the edge. The section of the web sample resting on the horizontal surface was held down by hand pressure. The test structure was large enough to allow the material to hang freely over the edge. Drapability was determined by measuring the shortest distance from the outer edge (of the bottom face) of the overhanging portion of the sample to the adjacent vertical surface of the test structure. The measured results for Examples 3 and 17 are recorded in Table 10.

**Table 10**

| | Measured Distance (mm) |
|---|---|
| Example 3 (unstretched) | 80 |
| Example 17 (stretched) | 25 |

The stretched web increased in drapability by 69% as compared to the unstretched web.

### Hysteresis

The hysteresis properties of the coiled nonwoven webs were measured using a universal constant rate of extension tensile testing instrument equipped with a computer for data recording and the required load ranges was used (Model 5500R available from Instron Engineering Corporation, Canton, MA). All testing was conducted at constant temperature (23°C ± 2°C) and relative humidity (50% ± 5%). All materials and equipment were equilibrated at these conditions for a minimum of 24 hours prior to testing. A 101.6 mm (CD) by 152.4 mm (MD) sample of web was mounted in the Instron instrument so that the sample was held with minimal slack when the jaws of the upper and lower jaws of the instrument were positioned at 76.2 mm apart. Line contact jaws were used to minimize slip and breakage of the sample in the jaws. The instrument crosshead speed was set to 305 mm/minute until a maximum extension of 76.2 mm (100% extension) was achieved. At 100 percent extension, the jaws were held stationary for one second and then returned to the zero extension position of 76.2 mm apart. In Table 11, the load value (in Newtons) measured at web extensions of 25%, 50%, 75%, and 100% are recorded for previously stretched coiled webs (Examples 15 and 19) and previously unstretched coiled webs (Examples 3 and 7). The data shows that previously stretched samples of web had significantly lower load at extensions of 25% when compared to the corresponding previously unstretched sample

**Table 11 - Load (N) at Percent Extension**

| | at 25% | At 50% | At 75% | At 100% |
|---|---|---|---|---|
| Example 3 (unstretched) | 16.6 | 18.9 | 17.4 | 18.9 |
| Example 19 (stretched) | 2.5 | 7.7 | 14.9 | 18.2 |
| Example 7(unstretched) | 20.9 | 23.3 | 25.4 | 25.6 |
| Example 15 (stretched) | 11.5 | 20.7 | 26.0 | 28.9 |

### Fluid Transport Rate

The rates of fluid transport by unstretched coiled webs (Examples 1, 3, 5-8, and 10) and stretched coiled webs (Examples 12 and 17-20) were measured with and without an applied compression force. A circular sample of the coiled web (75 mm diameter) was placed between two plexiglass plates (20.3 cm by 20.3 cm). A 15 mm diameter hole was cut through the center of the top plate. A glass, conical-shaped funnel (13 mm inner diameter stem) was positioned in the hole so that the bottom of the funnel stem was aligned flush with the bottom of the top plate. A minimum amount of Parafilm™ M (Pechiney Plastic Packaging, Chicago, IL) was wrapped around the stem in order to achieve a tight, non-leaking seal between the funnel stem and the hole in the plate. The top plate with attached funnel weighed 427 g. The coiled web sample was positioned so that the hole in the top plate was positioned directly above the center of the coiled web sample. To provide a compression force, individual weights were added to the top plate in a pattern that provided for an even distribution of the total weight (ranging from 0-24 added kilograms) on the top surface. The rate of fluid transport by the coiled webs under different compression forces (0-24 kg) was determined by quickly adding a saline solution (100 mL of 0.9% NaCl in water) to the funnel and measuring the time required for all of the fluid to enter the coiled web. To enhance visualization, the saline solution was dyed with red food coloring. The results for the coiled web samples prepared from Examples 1, 3, 5-8, 10, 12, and 17-20 are presented in Table 12.

**Table 12**

| | Fluid Transport Rate (seconds) with Weight Added (kg) | | | | | | | |
|---|---|---|---|---|---|---|---|---|
| | 0 kg | 1 kg | 4 kg | 8 kg | 12 kg | 16 kg | 20 kg | 24 kg |
| Example 1 | 2 | 3 | 4 | 4 | 5 | 5 | 6 | 7 |
| Example 3 | 3 | 3 | 3 | 4 | 6 | 6 | 7 | 8 |
| Example 5 | 2 | 2 | 2 | 3 | 3 | NT | NT | 12 |
| Example 6 | 3 | 2 | 2 | 3 | 3 | NT | NT | 3 |
| Example 7 | 2 | 3 | 4 | 7 | 8 | NT | NT | 12 |
| Example 8 | 2 | 2 | 2 | 2 | 2 | NT | NT | 2 |
| Example 10 | 2 | 2 | 2 | 2 | 2 | NT | NT | 3 |
| Example 12 | 2 | 2 | 6 | 8 | 8 | 10 | 11 | 12 |
| Example 17 | 2 | 4 | 8 | 11 | 11 | 13 | 15 | 16 |
| Example 18 | 3 | 3 | 6 | 9 | 14 | 12 | 12 | 16 |
| Example 19 | 2 | 3 | 6 | 9 | 10 | 12 | 13 | 13 |
| Example 20 | 2 | 2 | 3 | 5 | 7 | 8 | 8 | 9 |

| | | | | | | | | |
|---|---|---|---|---|---|---|---|---|
| NT - not tested | | | | | | | | |

### Fluid Uptake Rate and Fluid Distribution in Adult Incontinence Pads

The test apparatus and test method described above to measure the fluid transport rate of coiled web samples was used to measure the fluid uptake rates and the fluid distribution of adult incontinence pads that were modified by replacing a section of the absorbent core of the pad with a sample of coiled web. A central portion of the entire thickness of the absorbent core was removed and the coiled web inserted in place of the removed absorbent core, such that one major face of the web was in contact with the topsheet or acquisition-distribution layer (ADL), the opposing major surface of the web was in contact with the backsheet, and the lateral edges corresponding to the thickness of the web were in contact with the absorbent core. Examples of adult incontinence pads used as test articles are well known to those skilled in the art and have been previously described in U.S. Pat. No. 5,019,065 (Scripps), U.S. Pat. No. 6,509,513 (Glaug), U.S. Pat. No. 4,834,735 (Alemany), and U.S. Pat. No. 4,610,678 (Weisman). A typical adult incontinence pad test article was composed of a top sheet; bottom sheet; absorbent core element located between the top and bottom sheets; and an optional ADL located between the absorbent core element and the top sheet. The edges of the top sheet and bottom sheet were attached to form a seal. The overall dimensions of the test pads ranged from about 26-31 cm (length) by 9-11 cm (width) by 3-10 mm (thickness). The overall weight of the test pads ranged from about 11-26 g.

The top sheet was a liquid permeable polypropylene nonwoven having a basis weight of about 27-37 gsm. The back sheet was a liquid impermeable polyethylene film having a thickness of about 0.5-2.0 mil. The absorbent core material was composed of a mixture of cellulosic fibers (about 70-80% by weight) and superabsorbent polymer (about 20-30% by weight). The dimensions of the absorbent core element ranged from about 24-29 cm (length) by 7-9 cm (width) by 3-10 mm (thickness). The amount of absorbent core in the pads ranged from about 8-25 grams. The amount of absorbent core removed to insert the coiled web ranged from about 1.5-3.5 g. In examples of pads with an inserted coiled web component, the pad was reassembled but the opened edge regions were not resealed. The optional ADL was a tissue or nonwoven layer with a basis weight of about 50-150 gsm. The dimensions of the ADL ranged from about 17-31 cm (length) by 4-8 cm (width).

The incontinence pad was positioned in the test apparatus so that the topsheet of the pad faced the upper plate (backsheet resting on the lower plate) and the pad was centered with respect to the hole in the top glass plate. The amount of saline solution used in each test was 75 mL. The rate of fluid uptake by the pad under different compression forces (0-24 kg) was determined by quickly adding the saline solution to the funnel and measuring the time required for all of the fluid to enter the pad. The longitudinal distribution of the liquid in each pad was determined by removing the pad from the apparatus and measuring the total longitudinal distance (mm) traveled by the liquid in the pad. The leakage of liquid from the pads (grams) was measured by initially placing the entire test apparatus in a pan. Any liquid that leaked from the pad during the test was collected in the pan, recovered, and then weighed. The results are presented in Tables 13-17.

### Example 22

A TENA SERENITY adult incontinence pad (available from SCA Personal Products, Philadelphia, PA) was modified by removing by hand all of the absorbent core component in a 30 mm by 120 mm section of the pad (centered in both the longitudinal and lateral directions of the pad) and filling the subsequent void with a 1.16 g sample of Example 13. After filling the void, the pad was reassembled by repositioning the original top sheet and acquisition distribution layer (ADL) of the pad. In this configuration, the fluid delivered from the funnel passed through the top sheet and the ADL before flowing into the inserted coiled web (only a negligible amount of fluid contacted the absorbent core without first contacting the coiled web). The coiled web sample of Example 13 was reused for each of the test conditions. Between each test, the web sample was removed from a wet pad, dried by blotting, and then reinserted into the void of a fresh (dry) pad.

### Example 23

A POISE adult incontinence pad (available from Kimberly-Clark Corporation, Neenah, WI) was modified using the same procedure as described in Example 22. The void that was created in the pad was filled with a 1.09 g sample of Example 13.

### Example 24

A TENA LADY EXTRA adult incontinence pad (available from SCA Hygeine Products, Stockholm, Sweden) was modified by removing by hand all of the absorbent core component in a 25.4 mm by 140 mm section of the pad (centered in both the longitudinal and lateral directions of the pad) and filling the subsequent void with a 1.22 g sample of Example 15. After filling the void, the pad was reassembled by repositioning the original top sheet and acquisition distribution layer of the pad. In this configuration, the fluid delivered from the funnel passed through the top sheet and the ADL before flowing into the inserted coiled web (only a negligible amount of fluid contacted the absorbent core without first contacting the coiled web). The coiled web sample of Example 15 was reused for each of the test conditions. Between each test, the web sample was removed from a wet pad, dried by blotting, and then reinserted into the void of a fresh (dry) pad.

### Example 25

The same construction as in Example 22 was used with the exception that that the void in the pad was filled with a 1.94 g sample of Example 1, instead of Example 13.

### Example 26

The same construction as in Example 23 was used with the exception that that the void in the pad was filled with a 1.91 g sample of Example 1, instead of Example 13.

### Example 27

The same construction as in Example 24 was used with the exception that that the void in the pad was filled with a 1.68 g sample of Example 7, instead of Example 15.

### Comparative Examples B, C, and D

Comparative Example B was an unmodified TENA SERENITY adult incontinence pad. Comparative Example C was an unmodified POISE adult incontinence pad and Comparative Example D was an unmodified TENA LADY EXTRA adult incontinence pad.

**Table 13**

| | Fluid Uptake Rate (seconds) with Weight Added (kg) | | | | | | | |
|---|---|---|---|---|---|---|---|---|
| | 0 kg | 1 kg | 4 kg | 8 kg | 12 kg | 16 kg | 20 kg | 24 kg |
| Example 22 | 2 | 3 | 4 | 12 | 17 | 24 | 33 | 43 |
| Example 23 | 2 | 2 | 4 | 9 | 14 | 19 | 22 | 30 |
| Example 25 | 2 | 2 | 5 | 11 | 15 | 20 | 23 | 35 |
| Example 26 | 2 | 3 | 6 | 11 | 16 | 17 | 24 | 29 |
| Comparative Example B | 4 | 6 | 23 | 56 | 98 | 117 | 154 | 182 |
| Comparative Example C | 2 | 14 | 25 | 53 | 102 | 110 | 133 | 163 |

**Table 14**

| | Length of Longitudinal Fluid Distribution in the Pad (mm) with Weight Added (kg) | | | | | | | |
|---|---|---|---|---|---|---|---|---|
| | 0 kg | 1 kg | 4 kg | 8 kg | 12 kg | 16 kg | 20 kg | 24 kg |
| Example 22 | 140 | 155 | 160 | 169 | 166 | 170 | 173 | 170 |
| Example 23 | 130 | 150 | 142 | 143 | 185 | 175 | 175 | 160 |
| Example 25 | 180 | 190 | 160 | 200 | 200 | 205 | 210 | 173 |
| Example 26 | 155 | 180 | 150 | 175 | 190 | 190 | 180 | 170 |
| Comparative Example B | 71 | 75 | 85 | 80 | 85 | 85 | 90 | 100 |
| Comparative Example C | 73 | 75 | 85 | 83 | 78 | 85 | 82 | 95 |

**Table 15**

| | Leakage of Liquid from the Pad (grams) with Weight Added (kg) | | | | | | | |
|---|---|---|---|---|---|---|---|---|
| | 0 kg | 1 kg | 4 kg | 8 kg | 12 kg | 16 kg | 20 kg | 24 kg |
| Example 22 | 0 | 0 | 0 | 0 | 0 | 0 | 0 | 0 |
| Example 23 | 19.4 | 0.7 | 0 | 0 | 0 | 0 | 0 | 0 |
| Example 25 | 0 | 0 | 0 | 0 | 0 | 0 | 0 | 0 |
| Example 26 | 16.4 | 2.2 | 0 | 0 | 0 | 0 | 0 | 0 |
| Comparative Example B | 9.1 | 0 | 0 | 0 | 0 | 0 | 0 | 0 |
| Comparative Example C | 32.2 | 1.4 | 0.4 | 0 | 0 | 0 | 0 | 0 |

**Table 16**

| | Fluid Uptake Rate (seconds) with Weight Added (kg) | |
|---|---|---|
| | 4 kg | 24 kg |
| Example 24 | 3 | 32 |
| Example 27 | 3 | 33 |
| Comparative Example D | 31 | 102 |

**Table 17**

| | Length of Longitudinal Fluid Distribution in the Pad (mm) with Weight Added (kg) | |
|---|---|---|
| | 4 kg | 24 kg |
| Example 24 | 170 | 180 |
| Example 27 | 175 | 185 |
| Comparative Example D | 95 | 75 |

### Fluid Uptake Rate and Fluid Distribution in Adult Incontinence Pads Following Multiple Fluid Challenges.

Adult incontinence pads constructed according to Examples 22 and 25 were evaluated for fluid uptake rate and fluid distribution following two 75 mL saline solution challenges. The apparatus described above for determining fluid uptake rate was used. After the first challenge, the added weights and top glass plate were removed and the pad was maintained undisturbed for two minutes. The apparatus was reassembled, and the second saline solution challenge was made. The results are presented in Tables 18-19.

**Table 18**

| | Fluid Uptake Rate (seconds) with Weight Added (kg) Following Two Saline Solution Challenges | | | |
|---|---|---|---|---|
| | 0 kg | 1 kg | 4 kg | 12 kg |
| Example 22 | 2 | 4 | 8 | 31 |
| Example 25 | 23 | 3 | 10 | 29 |
| Comparative Example B | 23 | 41 | 97 | 262 |

**Table 19**

| | Length of Longitudinal Fluid Distribution in the Pad (mm) with Weight Added (kg) Following Two Saline Solution Challenges | | | |
|---|---|---|---|---|
| | 0 kg | 1 kg | 4 kg | 12 kg |
| Example 22 | 240 | 250 | 270 | 270 |
| Example 25 | 250 | 260 | 250 | 260 |
| Comparative Example B | 160 | 170 | 210 | 265 |

## Claims

1. An absorbent assembled intermediate comprising a thermoplastic nonwoven web proximate an absorbent material; wherein the thermoplastic nonwoven web comprises a plurality of coiled filaments, the filaments having an average diameter of 200 to 500 microns interengaged by means of intermittent bonds and the web has a thickness of 3 to 20 mm, a density ranging from 0.02 to 0.10 g/cm³, and a work of compression no greater than 20 kJ/m³ according to the compressibility test method.

2. The absorbent assembled intermediate of claim 1 wherein the coiled filaments are continuous in one direction.

3. The absorbent assembled intermediate of any one of claims 1-2 wherein the intermittent bonds comprise melt-bonds.

4. The absorbent assembled intermediate of any one of claims 1-3 wherein the web has a 15 basis weight ranging from 200 gsm to 400 gsm.

5. The absorbent assembled intermediate of any one of claims 1-4 wherein the web exhibits a
load of less than 25 newtons at 25% extension according to the hysteresis test method.

6. The absorbent assembled intermediate of any one of claims 1-5 wherein the nonwoven web exhibits a vertical wicking of saline solution of no greater than 5, 4, 3, 2, or 1 mm according to the vertical wicking test method.

7. The absorbent assembled intermediate of any one of claims 1-6 wherein the nonwoven web is free of hydrophilic fibers and superabsorbent polymer.

8. The absorbent assembled intermediate of any one of claims 1-7 wherein the nonwoven web exhibits any one or combination of fluid transport rate properties according to the fluid
transport rate test method selected from
a fluid transport rate of no greater than 10 seconds with a weight of 4 kg;
a fluid transport rate of no greater than 15 seconds with a weight of 12 kg; and
a fluid transport rate of no greater than 20 seconds with a weight of 24 kg.

9. The absorbent assembled intermediate of any one of claims 1-8 wherein the nonwoven web is a stretched web and a face of the nonwoven web comprises at least 1, 2, or 3 broken bonds per cm².

10. The absorbent assembled intermediate of claims 1-9 wherein the nonwoven web comprises a first major face and a second opposing major face substantially parallel to the first major face and a thickness in a direction orthogonal to the first and second major face;
wherein the thickness define lateral edges and the absorbent material is in contact with at least one lateral edge.

11. The absorbent assembled intermediate of claim 10 wherein at least two opposing lateral
edges of the non woven web are in contact with the absorbent material and two opposing lateral edges of the non woven are fluid impervious.

12. The absorbent assembled intermediate of any one of claims 1-11 wherein
the
absorbent component exhibits any one or combination of fluid uptake rate properties according to the fluid uptake rate test selected from
a fluid uptake rate of no greater than 10 seconds with a weight of 4 kg;
a fluid uptake rate of no greater than 50 seconds with a weight of 12 kg; and
a fluid uptake rate of no greater than 50 seconds with a weight of 24 kg.

13. An absorbent article comprising the absorbent assembled intermediate of any one of claims 1-12, wherein the article is not a personal hygiene article.

14. The absorbent article of claim 13, wherein the article is a wound dressing.

15. The absorbent article of claim 13, wherein the article is for medical use.

16. The absorbent article of claim 13, wherein the absorbent article is for spill containment.

17. The absorbent assembled intermediate of any one of claims 1-12, wherein the filament cross sectional dimension and shape of the cross-section is substantially uniformly round along the length of the filament.

## Patentansprüche

1. Absorbierendes zusammengesetztes Zwischenprodukt, aufweisend eine thermoplastische Vliesbahn in der Nähe eines absorbierenden Materials; wobei die thermoplastische Vliesbahn mehrere gewickelte Filamente aufweist, wobei die Filamente einen mittleren Durchmesser von 200 bis 500 Mikron aufweisen, die durch intermittierende Bindungen miteinander in Eingriff stehen, und die Bahn eine Dicke von 3 bis 20 mm, eine Dichte im Bereich von 0,02 bis 0,10 g/cm³ und eine Kompressionsarbeit gemäß dem Kompressibilitätsprüfverfahren von nicht mehr als 20 kJ/m³ aufweist.

2. Absorbierendes zusammengesetztes Zwischenprodukt nach Anspruch 1, wobei die gewickelten Filamente in einer Richtung kontinuierlich sind.

3. Zusammengesetztes absorbierendes Zwischenprodukt nach einem der Ansprüche 1 bis 2, wobei die intermittierenden Bindungen Schmelzbindungen umfassen.

4. Zusammengesetztes absorbierendes Zwischenprodukt nach einem der Ansprüche 1 bis 3, wobei die Bahn ein Basisgewicht im Bereich von 200 gsm bis 400 gsm aufweist.

5. Zusammengesetztes absorbierendes Zwischenprodukt nach einem der Ansprüche 1 bis 4, wobei die Bahn bei einer Dehnung von 25 % gemäß dem Hystereseprüfverfahren eine Last von weniger als 25 Newton aufweist.

6. Zusammengesetztes absorbierendes Zwischenprodukt nach einem der Ansprüche 1 bis 5, wobei die Vliesbahn gemäß dem Prüfverfahren zur vertikalen Dochtwirkung eine vertikale Dochtwirkung einer Salzlösung von nicht mehr als 5, 4, 3, 2 oder 1 mm zeigt.

7. Zusammengesetztes absorbierendes Zwischenprodukt nach einem der Ansprüche 1 bis 6, wobei die Vliesbahn frei von hydrophilen Fasern und superabsorbierendem Polymer ist.

8. Zusammengesetztes absorbierendes Zwischenprodukt nach einem der Ansprüche 1 bis 7, wobei die Vliesbahn gemäß dem Fluidtransportraten-Prüfverfahren eine beliebige oder eine Kombination von Fluidtransportrateneigenschaften aufweist, die ausgewählt sind aus
einer Fluidtransportrate von nicht mehr als 10 Sekunden bei einem Gewicht von 4 kg;
einer Fluidtransportrate von nicht mehr als 15 Sekunden bei einem Gewicht von 12 kg; und
einer Fluidtransportrate von nicht mehr als 20 Sekunden bei einem Gewicht von 24 kg.

9. Zusammengesetztes absorbierendes Zwischenprodukt nach einem der Ansprüche 1 bis 8, wobei die Vliesbahn eine gestreckte Bahn ist und eine Fläche der Vliesbahn mindestens 1, 2 oder 3 gebrochene Bindungen pro cm² umfasst.

10. Zusammengesetztes absorbierendes Zwischenprodukt nach den Ansprüchen 1 bis 9, wobei die Vliesbahn eine erste Hauptfläche und eine zweite gegenüberliegende Hauptfläche, die zur ersten Hauptfläche im Wesentlichen parallel ist, und eine Dicke in einer zur ersten und zur zweiten Hauptfläche orthogonalen Richtung aufweist;
wobei die Dicke Seitenkanten definiert und das absorbierende Material mit mindestens einer Seitenkante in Kontakt steht.

11. Zusammengesetztes absorbierendes Zwischenprodukt nach Anspruch 10, wobei mindestens zwei gegenüberliegende Seitenkanten der Vliesbahn mit dem absorbierenden Material in Kontakt stehen und zwei gegenüberliegende Seitenkanten des Vlieses fluidundurchlässig sind.

12. Zusammengesetztes absorbierendes Zwischenprodukt nach einem der Ansprüche 1 bis 11, wobei der absorbierende Bestandteil gemäß der Fluidaufnahmeraten-Prüfung eine beliebige oder eine Kombination von Fluidaufnahmerateneigenschaften zeigt, die ausgewählt sind aus
einer Fluidaufnahmerate von nicht mehr als 10 Sekunden bei einem Gewicht von 4 kg;
einer Fluidaufnahmerate von nicht mehr als 50 Sekunden bei einem Gewicht von 12 kg; und
einer Fluidaufnahmerate von nicht mehr als 50 Sekunden bei einem Gewicht von 24 kg.

13. Absorptionsartikel, der das absorbierende zusammengesetzte Zwischenprodukt nach einem der Ansprüche 1 bis 12 aufweist, wobei der Artikel kein Körperhygieneartikel ist.

14. Absorptionsartikel nach Anspruch 13, wobei der Artikel ein Wundverband ist.

15. Absorptionsartikel nach Anspruch 13, wobei der Artikel zur medizinischen Verwendung bestimmt ist.

16. Absorptionsartikel nach Anspruch 13, wobei der Absorptionsartikel zur Überlaufeindämmung dient.

17. Zusammengesetztes absorbierendes Zwischenprodukt nach einem der Ansprüche 1 bis 12, wobei die Filamentquerschnittsabmessung und die Form des Querschnitts entlang der Länge des Filaments im Wesentlichen gleichmäßig rund ist.

## Revendications

1. Intermédiaire assemblé absorbant comprenant une bande non tissée thermoplastique à proximité d'un matériau absorbant ; dans lequel la bande non tissée thermoplastique comprend une pluralité de filaments enroulés, les filaments ayant un diamètre moyen de 200 à 500 micromètres en contact mutuel au moyen de liaisons intermittentes et la bande a une épaisseur de 3 à 20 mm, une masse volumique allant de 0,02 à 0,10 g/cm³, et un travail de compression n'excédant pas 20 kJ/m³ selon le procédé de test de compressibilité.

2. Intermédiaire assemblé absorbant selon la revendication 1, dans lequel les filaments enroulés sont continus dans une direction.

3. Intermédiaire assemblé absorbant selon l'une quelconque des revendications 1 à 2, dans lequel les liaisons intermittentes comprennent des liaisons par fusion.

4. Intermédiaire assemblé absorbant selon l'une quelconque des revendications 1 à 3, dans lequel la bande a une masse surfacique allant de 200 g/m² à 400 g/m².

5. Intermédiaire assemblé absorbant selon l'une quelconque des revendications 1 à 4, dans lequel la bande présente une charge inférieure à 25 newtons à une extension de 25 % selon le procédé de test d'hystérésis.

6. Intermédiaire assemblé absorbant selon l'une quelconque des revendications 1 à 5, dans lequel la bande non tissée présente une capillarité verticale de solution saline n'excédant pas 5, 4, 3, 2 ou 1 mm selon le procédé de test de capillarité verticale.

7. Intermédiaire assemblé absorbant selon l'une quelconque des revendications 1 à 6, dans lequel la bande non tissée est exempte de fibres hydrophiles et de polymère superabsorbant.

8. Intermédiaire assemblé absorbant selon l'une quelconque des revendications 1 à 7, dans lequel la bande non tissée présente l'une quelconque ou une combinaison de propriétés de vitesse de transport de fluide selon le procédé de test de vitesse de transport de fluide, choisie parmi
une vitesse de transport de fluide n'excédant pas 10 secondes avec un poids de 4 kg ;
une vitesse de transport de fluide n'excédant pas 15 secondes avec un poids de 12 kg ; et
une vitesse de transport de fluide n'excédant pas 20 secondes avec un poids de 24 kg.

9. Intermédiaire assemblé absorbant selon l'une quelconque des revendications 1 à 8 dans lequel la bande non tissée est une bande étirée et une face de la bande non tissée comprend au moins 1, 2, ou 3 liaisons rompues par cm².

10. Intermédiaire assemblé absorbant selon les revendications 1 à 9, dans lequel la bande non tissée comprend une première face principale et une deuxième face principale opposée essentiellement parallèle à la première face principale et une épaisseur dans une direction orthogonale aux première et deuxième faces principales ;
dans lequel l'épaisseur définit des bords latéraux et le matériau absorbant est en contact avec au moins un bord latéral.

11. Intermédiaire assemblé absorbant selon la revendication 10, dans lequel au moins deux bords latéraux opposés de la bande non tissée sont en contact avec le matériau absorbant et deux bords latéraux opposés de la bande non tissée sont imperméables aux fluides.

12. Intermédiaire assemblé absorbant selon l'une quelconque des revendications 1 à 11, dans lequel le composant absorbant présente l'une quelconque ou une combinaison de propriétés de vitesse d'absorption de fluide selon le test de vitesse d'absorption de fluide, choisie parmi
une vitesse d'absorption de fluide n'excédant pas 10 secondes avec un poids de 4 kg ;
une vitesse d'absorption de fluide n'excédant pas 50 secondes avec un poids de 12 kg ; et
une vitesse d'absorption de fluide n'excédant pas 50 secondes avec un poids de 24 kg.

13. Article absorbant comprenant l'intermédiaire assemblé absorbant selon l'une quelconque des revendications 1 à 12, où l'article n'est pas un article d'hygiène personnelle.

14. Article absorbant selon la revendication 13, où l'article est un pansement pour plaie.

15. Article absorbant selon la revendication 13, où l'article est pour usage médical.

16. Article absorbant selon la revendication 13, où l'article absorbant est destiné au confinement de déversements.

17. Intermédiaire assemblé absorbant selon l'une quelconque des revendications 1 à 12, dans lequel la dimension en coupe transversale de filament et la forme de la coupe transversale sont arrondies de façon essentiellement uniforme sur la longueur du filament.
